# FASCICULE DE BREVET EUROPEEN

(11) **EP 0 796 338 B1**
(45) Date de publication et mention de la délivrance du brevet: **31.05.2006**
(21) Numéro de dépôt: 95941756.9
(22) Date de dépôt: 30.11.1995
(51) Int. Cl.: C12N 15/86, C07K 14/15, C12N 5/10, A61K 48/00, C12N 7/04

(54) **LIGNEES CELLULAIRES D'ENCAPSIDATION POUR LA TRANSCOMPLEMENTATION DE VECTEURS RETROVIRAUX DEFECTIFS**
VERPACKUNGSZELLINIE ZUR TRANSKOMPLEMENTIERUNG VON DEFEKTIVEN RETROVIREN-VEKTOREN
ENCAPSIDATION CELL LINES FOR THE TRANSCOMPLEMENTATION OF DEFECTIVE RETROVIRAL VECTORS

(30) Priorité: 30.11.1994 FR 9414406
(43) Date de publication de la demande: 24.09.1997
(73) Titulaire: Cohen-Haguenauer, Odile, F-75116 Paris (FR)
(72) Inventeur: Cohen-Haguenauer, Odile, F-75116 Paris (FR)
(74) Mandataire: Breese, Pierre
(86) Numéro de dépôt international: PCT/FR1995/001591
(87) Numéro de publication internationale: WO 1996/017071

(56) Documents cités:
- EP-A- 0 611 822
- EP-A- 0 632 129
- WO-A-90/02797
- WO-A-92/05266
- FR-A- 2 707 091
- US-A- 4 650 764
- US-A- 5 124 263
- VIROLOGY, vol. 207, no. 1, 20 Février 1995 ORLANDO US, pages 271-275, RONFORT, C. ET AL. 'Defective retroviral endogenous RNA is efficiently transmitted by infectious particles produced on an avian retroviral vector packaging cell line'
- POULTRY SCIENCE, vol. 74, 1995 pages 127-135, RONFORT, C. ET AL. 'Structure and expression of endogenous retroviral sequences in the permanent LMH Chicken cell line'
- VIROLOGY, vol. 208, no. 1, 1 Avril 1995 ORLANDO US, pages 234-241, MARTINEZ, I. & DORNBURG, R. 'Improved retroviral packaging lines derived from Spleen Necrosis Virus'
- JOURNAL OF VIROLOGY, vol. 64, no. 3, Mars 1990 WASHINGTON D.C., USA, pages 1070-1078, COSSET, F.L. ET AL. 'A new Avian Leukosis Virus-based packaging cell line that uses two separate transcomplementing helper genomes'
- JOURNAL OF VIROLOGY, vol. 69, no. 12, Décembre 1995 WASHINGTON, D.C., USA, pages 7430-7436, COSSET, F.L. ET AL. 'High-titer packaging cells producing recombinant retroviruses resistant to human serum'
- J MOL MED, vol. 73, 1995 pages 113-120, HOATLIN, M.E. ET AL. 'Amplified and tissue-directed expression of retroviral vectors using ping-pong techniques'

## Description

L'invention a pour objet de nouvelles lignées cellulaires dites lignées de transcomplémentation permettant l'encapsidation d'ARN rétroviraux recombinants portant des séquences de nucléotides issues de gènes présentant par exemple un intérêt thérapeutique (appelés transgènes) pour le transfert et l'expression de ces transgènes dans des cellules cibles eucaryotes. L'invention concerne aussi des vecteurs d'expression pour la transcomplémentation de vecteurs rétroviraux défectifs.

Le transfert de gènes à visée thérapeutique ou "thérapie génique" somatique consiste à insérer un gène "réparateur" (transgène) dans les cellules somatiques d'un organisme constitué, pour pallier au dysfonctionnement d'un gène endogène ; voire y ajouter une fonction nouvelle dans un but thérapeutique. Le changement génétique résultant est susceptible de se transmettre aux cellules filles de la cellule manipulée, mais il ne sera pas héréditaire. La contrepartie normale de séquences d'ADN altérées se voit ainsi transformée en médicament.

Différentes approches sont actuellement explorées pour introduire les gènes ou séquences nucléotidiques en général à visée thérapeutique dans les cellules cibles. Ces cellules cibles peuvent être les cellules à traiter, ou des cellules intermédiaires entre le vecteur du transgène et les cellules à traiter.

Les vecteurs actuellement utilisés pour le transfert de gènes dans des cellules cibles, sont dérivés soit de virus inactivés, tels que les rétrovirus ou les adénovirus, soit de complexes macromoléculaires. Les rétrovirus s'adressent souvent à un tissu cible comprenant un contingent de cellules souches susceptibles d'être manipulées ex vivo ; par contre, lorsque le tissu cible est constitué de cellules en différenciation terminale, ou bien intriqué dans un organe dont les contraintes architecturales ont des conséquences fonctionnelles majeures, comme le poumon, le transfert de gènes doit s'opérer in vivo, par exemple au moyen d'adénovirus.

La thérapie génique trouve ses applications dans des pathologies aussi diverses que les maladies héréditaires dues à l'altération d'un seul gène, comme la myopathie de Duchenne, les maladies lysosomiales, la mucoviscidose ou des maladies acquises telles que le sida, les cancers, la maladie thrombo-embolique ou des maladies neurologiques dégénératives, les maladies hématologiques constitutionnelles.

Le développement de vecteurs rétroviraux plus efficaces que les outils existant constitue un objectif prioritaire. En effet, les vecteurs rétroviraux ont fait la preuve de leur efficacité pour transférer des gènes de façon stable et permanente à des cellules cibles ; qui sont l'objet de mitoses et comportent idéalement un contingent de cellules souches.

Les limitations sont essentiellement liées à une infectivité insuffisante des virus utilisés et/ou un niveau de transcription trop modeste. D'autres limitations potentielles tiennent aux conditions de sécurité du transfert qui demeurent actuellement à un niveau qui n'est pas satisfaisant ; tant par le risque de génération de virus compétents pour la réplication que du fait de la transmission conjointe de séquences endogènes encapsidables générées par les lignées d'encapsidation permettant la complémentation.

Il importe donc de développer des systèmes vectoriels plus performants et de plus haute sécurité que ceux existants.

Lorsque le transgène à exprimer est administré au moyen d'un vecteur rétroviral, une étape nécessaire précédant l'infection des cellules cibles comprend la préparation de stocks de particules virales vectrices contenant le transgène.

Pour réaliser cette étape, on a recours à une transcomplémentation du vecteur rétroviral défectif portant sur le transgène, par les protéines virales dont les séquences ont été excisées pour introduire le transgène. Cette transcomplémentation peut être réalisée dans des cellules d'encapsidation. Ces cellules sont des cellules recombinantes comprenant le matériel génétique nécessaire à la synthèse des constituants rétroviraux, en particulier des protéines d'enveloppe ENV, des nucléoprotéines GAG et de la transcriptase inverse (ou réverse transcriptase) POL, nécessaire à la réplication virale. Les gènes gag, pol et env sont transfectés dans les cellules d'encapsidation par transfection des séquences d'acide nucléique qui les contiennent, au moyen d'un vecteur de transcomplémentation défectif pour la séquence Psi nécessaire à l'encapsidation virale.

L'expression dans les cellules d'encapsidation, des gènes gag, pol et env doit permettre l'encapsidation ultérieure de séquences d'ARN rétroviral recombinées avec la séquence transgénique d'intérêt, portées par un vecteur rétroviral comportant la séquence d'encapsidation Psi. L'encapsidation peut avoir lieu après transcomplémentation par les protéines virales codées par les séquences gag, pol, et env.

Les lignées d'encapsidation actuellement disponibles et dérivées de cellules murines, présentent les inconvénients suivants :
1) Co-encapsidation, concomitante des constructions défectives, de séquences rétrovirales endogènes (MCF, VL30, voire rétro-transposons). Ces séquences co-encapsidées sont donc susceptibles elles aussi de s'intégrer, après infection des cellules cibles du transfert.
2) L'expression de protéines de complémentation est pilotée dans ces lignées d'encapsidation, par un LTR rétroviral. Bien que les lignées de troisième génération, utilisent des constructions rétrovirales de complémentation, comprenant plusieurs sites de mutation ou délétion, le maintien de séquences LTR peut représenter un inconvénient potentiel ; en effet, ces séquences sont susceptibles de donner lieu à une recombinaison génétique avec les constructions défectives à complémenter.
   Ceci a pu être observé même dans les lignées dites de 3ème génération, où les séquences codant pour les protéines GAG et POL, sont transfectées séparément des séquences codant pour l'enveloppe afin de minimiser la probabilité des combinaisons génératrices de virus compétent.
   Par ailleurs, les séquences d'enveloppe actuellement utilisées dans la complémentation des virus défectifs sont des enveloppes amphotropes lorsque l'on s'adresse au transfert dans les cellules de mammifères et en particulier de primates et d'hommes. Il n'est cependant pas certain que les récepteurs aux virus amphotropes soient assez représentés dans toutes les espèces cellulaires que l'on souhaite infecter dans une optique de thérapie génique.
   L'ensemble de ces éléments engagent à tenter d'améliorer la sécurité des conditions de transfert génétique avec des vecteurs rétroviraux.
   L'invention a pour objet de fournir des moyens pour la préparation de nouvelles lignées d'encapsidation remédiant au moins en partie à un ou plusieurs des problèmes précédemment décrits.
   Les lignées de l'invention permettent d'améliorer la sécurité du transfert des transgènes, puisqu'elles sont développées à partir de cellules d'espèces animales dépourvues de rétrovirus endogènes identifiés à ce jour, et en mettant en oeuvre de façon préférée des promoteurs non viraux pour diriger l'expression d'au moins certaines des protéines de complémentation, et en particulier de promoteurs inductibles pour la synthèse conditionnelle des protéines de complémentation des vecteurs défectifs et notamment de l'enveloppe, à demande, lorsqu'un surnageant viral infectieux est en préparation.
   En outre, la complémentation pour les protéines GAG et POL est obtenue en utilisant les séquences de nucléotides d'un rétrovirus de Friend à partir duquel un vecteur a été construit.
   Pour réaliser l'invention, les séquences gag et pol sont transfectées séparément des séquences d'enveloppe dans la lignée cellulaire destinée à l'encapsidation.
   Ces lignées conduisent aussi à une amélioration de l'efficacité du transfert en utilisant des séquences d'enveloppe, en particulier d'origine humaine, afin de contourner le problème éventuel de l'insuffisance de l'expression du récepteur à un virus amphotrope à la surface de cellules humaines cibles de thérapie génique ; en particulier potentiellement lorsque les cellules cibles sont des cellules souches hématopoïétiques.
   L'invention a en premier lieu pour objet un vecteur d'expression pour la transcomplémentation d'un vecteur rétroviral qui permet le transfert et/ou l'intégration au sein du génome d'une cellule cible, d'une séquence de nucléotides choisie (dite "séquence transgénique"), caractérisé en ce qu'il comprend :
   a) une séquence de nucléotides dite séquence env, codant pour les polypeptides dérivés de l'enveloppe (polypeptide ENV) d'un rétrovirus de la famille des spumavirus, les polypeptides ENV permettant l'encapsidation d'ARN rétroviraux,
   b) des signaux régulateurs de transcription contrôlant l'expression de la séquence env,
   ledit vecteur d'expression étant dépourvu du signal d'encapsidation.

Les rétrovirus de la famille des spumavirus sont des rétrovirus que l'on peut isoler dans des cultures de cellules humaines ou animales.

Un rétrovirus de la famille des spumavirus, dénommé HSRV a été isolé chez un patient présentant un carcinome nasopharyngé en 1971 (Hachong et al, J. Natl. Conser. Inst. 46, 299-307). Le rétrovirus HSRV a été cloné et séquencé et sa séquence a été décrite dans la publication de Flugel RM et al (THE EMBO JOURNAL, vol. 6, n° 7, pages 2077-2084, 1987). En particulier, cette publication décrit la séquence d'enveloppe du rétrovirus HSRV.

La séquence d'enveloppe du rétrovirus HSRV utilisée dans le cadre de la présente invention présente l'avantage tout à fait intéressant de coder pour une glycoprotéine susceptible d'être reconnue par un grand nombre de récepteurs à la surface des cellules humaines, et en particulier une glycoprotéine disposant de récepteurs au sein de la population de cellules humaines, plus nombreux que les récepteurs reconnaissant les glycoprotéines d'enveloppes amphotropes. La séquence d'enveloppe utilisée dans le cadre de la présente invention a avantageusement un tropisme spécifiquement humain ou de primate, pour le transfert de gènes chez l'homme ou le primate.

En particulier, la séquence env de spumavirus a un tropisme intéressant pour les cellules précurseurs hématopoiétiques humains.

Outre les cellules souches hématopoiétiques, les cellules primaires humaines telles que des fibroblastes et des lymphocytes peuvent être ciblées avec succès (infection lytique). Une infection chronique par un virus défectif a aussi été mise en évidence chez l'homme dans un tissu épithélial glandulaire et du tissu musculaire. Des lignées d'origine humaine épithéliales et lymphocytaires ainsi que les cellules HeLa sont également susceptibles de constituer une cible. Chez le lapin, toutes les cellules primaires testées sont infectables. Le spectre d'infection est donc très large.

La séquence env présente dans le vecteur d'expression pour la transcomplémentation peut être placée pour son expression sous le contrôle de signaux régulateurs de transcription rétroviraux. En particulier, ces signaux régulateurs de transcription peuvent être issus de la séquence LTR de rétrovirus tels que le rétrovirus de Friend.

Dans le cadre de la présente invention, la souche utilisée du virus de Friend est une souche particulièrement virulente. L'isolat 1-5 du virus écotrope de la leucémie murine de Friend a été obtenu à partir de cultures de moelle à long terme infectées par le complexe viral inducteur de polyglobulies du virus de Friend (FV-P) (Mathieu-Mahul et al., Virology, 119: 59-67, 1982). La souche FB29 du F-MuLV dérivée de l'isolat I-5 (Sitbon et al, Cell, 47: 851-859, 1986) est responsable d'effets cytolytiques et leucémogènes sur les cellules érythroïdes, conduisant à une anémie hémolytique précoce et sévère suivie d'une érythroleucémie tardive chez les souris susceptibles, inoculées à la naissance. Les régions responsables de l'érythroleucémie ont été localisées au niveau de la région U3 du LTR viral (Sitbon et al, 1986 ; Sitbon et al, PNAS USA, 88: 5932-5936, 1991). Le déterminant principal de l'anémie hémolytique semble dépendre de séquences d'enveloppe spécifiques de la souche FB29 ; la sévérité pouvant en être influencée par trois régions distinctes, dont un segment structural de l'enveloppe, des séquences activatrices de la transcription localisées dans la région U3, et enfin, des séquences des régions U5-gag-pol (Sitbon et al., J. Virol. 64: 2135-2140, 1990). En outre, des analyses en microscopie électronique des particules virales ont authentifié une capacité d'encapsidation significativement supérieure (1,5 à 2 log).

Ainsi, la séquence env du rétrovirus de type spumavirus peut être placée sous le contrôle de la séquence LTR ou d'une partie de la séquence LTR de la souche FB29 du virus de Friend, suffisante pour assurer la transcription de la séquence de nucléotides env.

Dans un autre mode de réalisation de l'invention, le vecteur d'expression pour la transcomplémentation est caractérisé en ce que les signaux régulateurs de transcription de la séquence env comprennent un promoteur non viral. Le cas échéant, ce promoteur est suivi de la séquence de polyadénylation du virus SV40 ou d'une autre séquence de polyadénylation.

L'utilisation d'un promoteur non viral améliore les qualités de sécurité du vecteur de transcomplémentation ainsi construit.

En outre, lorsque le promoteur utilisé est un promoteur inductible, c'est à dire activable par une molécule donnée, en particulier un agent pharmacologique , l'expression de la séquence env peut être déclenchée à la demande.

De même, les séquences gag et pol transfectées séparément peuvent être placées sous le contrôle d'un promoteur inductible.

Alternativement, les signaux de transcription contrôlant l'expression de la séquence env et/ou l'expression des séquences gag et pol, peuvent comporter un promoteur conditionnel capable de s'exprimer uniquement dans certains tissus.

Un promoteur inductible approprié pour la réalisation de l'invention est par exemple le promoteur du récepteur β à l'acide rétinoique (promoteur RARβ).

Ce promoteur a été décrit par De Thé H. et al, dans la publication Nature, vol. 49, n° 6254, pages 177-180 datée du 11 Janvier 1990.

Un vecteur d'expression préféré pour la transcomplémentation est tel que la séquence env du rétrovirus de la famille des spumavirus est sous le contrôle d'un fragment HindIII-BamHI du récepteur β à l'acide rétinoïque tel que décrit dans la publication précitée de De Thé et al.

Selon un autre aspect de l'invention, le vecteur d'expression pour la transcomplémentation en enveloppe est caractérisé en ce qu'il comprend
a) une séquence de nucléotides dite séquence env, codant pour les polypeptides dérivés d'une enveloppe par exemple amphotrope, par exemple l'enveloppe 4070A du virus de la leucémie murine de Moloney (Mo-MuLV),
b) des signaux régulateurs de transcription non viraux, contrôlant l'expression de la séquence env, contenant par exemple un promoteur inductible ou conditionnel, par exemple le promoteur RAR-β.

Selon un mode de réalisation particulier de l'invention, le vecteur d'expression pour la transcomplémentation en enveloppe est caractérisé en ce que la séquence de nucléotides du vecteur d'expression pour la transcomplémentation, codant pour les polypeptides d'enveloppe est modifiée, par exemple par remplacement de nucléotides par un enchainement de nucléotides codant pour un polypeptide ou une glycoprotéine reconnue spécifiquement par un type cellulaire défini, ou par l'addition d'un tel enchainement.

Selon une variante de réalisation, l'invention a aussi pour objet un vecteur d'expression pour la transcomplémentation d'un vecteur rétroviral permettant le transfert et/ou l'intégration au sein du génome d'une cellule cible, d'une séquence de nucléotides choisie (dite "séquence transgénique"), comprenant :
a) une séquence de nucléotides dite séquence gag codant pour les polypeptides dérivés d'une nucléoprotéine (polypeptides GAG) d'un rétrovirus du type du rétrovirus de Friend,
b) une séquence de nucléotides dite séquence pol codant pour les polypeptides dérivés dont une protéine transcriptase inverse et une intégrase (polypeptides POL) d'un rétrovirus du type du rétrovirus de Friend,
c) des signaux régulateurs de transcription non viraux contrôlant l'expression des séquences gag et pol, ces signaux contenant par exemple un promoteur inductible ou conditionnel, par exemple le promoteur RAR-β et le susdit vecteur d'expression étant dépourvu de signaux d'encapsidation.

Les vecteurs d'expression pour la transcomplémentation selon l'invention peuvent être de type intégratif naturellement ou après sélection ou au contraire, il peut s'agir de vecteurs épisomals.

La présente demande a également pour objet une cellule eucaryote pour l'encapsidation par transcomplémentation, d'ARN rétroviraux recombinants portés par un vecteur rétroviral qui permet le transfert et l'intégration au sein du génome d'une cellule cible, d'une séquence de nucléotides choisie (séquence transgénique), ladite cellule étant caractérisée en ce qu'elle vérifie les propriétés suivantes:
- elle est sélectionnée parmi les cellules d'espèces animales dépourvues de rétrovirus endogènes, de préférence choisies parmi les cellules foetales ou embryonnaires, notamment de chien ou de lapin,
- elle a une morphologie homogène et stable dans le temps,
- elle n'est pas d'origine tumorale,
- elle a la capacité d'être sélectionnée dans un milieu de culture minimum dépourvu de CO₂, sans transformation préalable,
- elle a un taux de multiplication rapide.

Selon l'invention, l'expression "sans transformation préalable" signifie que la cellule ne bénéficie pas, avant sélection, d'un apport de séquence oncogénique.

La morphologie est homogène et stable dans le temps, dès lors qu'elle n'est pas substantiellement altérée pendant une durée de 6 mois ou plus.

Selon un mode de réalisation avantageux de l'invention, une cellule eucaryote pour l'encapsidation par transcomplémentation, d'ARN rétroviraux recombinants portés par un vecteur rétroviral qui permet le transfert et l'intégration au sein du génome d'une cellule cible, d'une séquence de nucléotides choisie (séquence transgénique), sélectionnée parmi les cellules d'espèces animales dépourvues de rétrovirus endogènes, de préférence choisies parmi les cellules foetales ou embryonnaires, notamment de chien ou de lapin, est susceptible d'être obtenue par sélection conformément à la méthode suivante :
a) culture de cellules eucaryotes choisies sur milieu riche ISCOVE (GIBCO) contenant en outre 10% de sérum de veau foetal et 10% de sérum de cheval,
b) passage des cellules cultivées en milieu DMEM contenant 20% de sérum de veau foetal et culture des cellules pendant un mois dans un milieu sans CO₂,
c) sélection des cellules ayant un taux de multiplication rapide (cinétique accélérée),
d) passage des cellules sélectionnées en milieu DMEM contenant 10% de sérum de veau foetal,
e) passage des cellules récupérées à l'étape d) en milieu DMEM contenant 10% de sérum de veau nouveau-né (HyClone),
f) récupération des cellules sélectionnées à l'issue de l'étape e).

Une cellule eucaryote peut ainsi être constituée par la cellule foetale de chien désignée par l'expression DOGOM 1, déposée à la Collection Nationale de Cultures de Microorganismes (CNCM) à Paris, France le 30 novembre 1994 sous le numéro 1-1496.

La cellule foetale de chien ainsi obtenue, répond de façon tout à fait satisfaisante aux critères de sélection suivants :
- elle est dépourvue de rétrovirus endogènes, il s'agit d'une cellule dont les propriétés d'adhérence sont satisfaisantes, elle présente une croîssance rapide, un morphologie stable et homogène ; cette cellule foetale de chien est également aisément transfectable et peut supporter un nombre de passages très élevés (passages artificiels intensifs pour tests) et est capable de soutenir un test LTC-IC (Long Term Culture Initiating Cells) tel que décrit dans la partie expérimentale.

A partir des cellules ainsi sélectionnées, des cellules pour l'encapsidation d'ARN rétroviraux sont préparées par modification génétique pas exemple par transfection dans les cellules ci-dessus décrites, ou infection de ces dernières avec les séquences de nucléotides codant pour les polypeptides ou les glycoprotéines de transcomplémentation, lesdites séquences étant portées par au moins deux vecteurs, voire au moins trois vecteurs. On a par exemple recours à, d'une part, un vecteur par exemple d'un plasmide-vecteur ou un vecteur rétroviral pour la transcomplémentation capable d'exprimer la séquence de nucléotides env codant pour les polypeptides d'enveloppe d'un rétrovirus de la famille des spumavirus et, d'autre part, à un vecteur, par exemple un plasmide-vecteur d'expression ou un vecteur rétroviral pour la transcomplémentation des séquences de nucléotides gag et pol d'un rétrovirus du type du rétrovirus de Friend.

Selon un autre mode de réalisation de l'invention, les cellules transcomplémentantes sont des cellules xénogéniques, allogéniques ou autologues susceptibles de permettre la libération in vivo chez un patient, d'un vecteur défectif infectieux. Dans ce cas, les cellules transcomplémentantes peuvent être les cellules cibles de l'infection par le vecteur rétroviral. A titre d'exemple, on utilisera les cellules endothéliales, musculaires ou stromales.

La présente demande concerne une cellule recombinante permettant l'encapsidation d'ARNs rétroviraux recombinants par transcomplémentation (cellules recombinantes transcomplémentantes), caractérisée en ce qu'il s'agit d'une cellule telle que définie précédemment, génétiquement modifiée, par exemple transfection ou par infection avec :
- d'une part, un premier vecteur d'expression pour la transcomplémentation d'un vecteur rétroviral permettant le transfert et/ou l'intégration au sein du génome d'une cellule cible d'une séquence de nucléotides choisie (dite "séquence transgénique"), comprenant les séquences complémentantes suivantes :
   a) une séquence de nucléotides dite séquence gag codant pour les polypeptides dérivés d'une nucléoprotéine (polypeptides GAG) d'un rétrovirus du type du rétrovirus de Friend,
   b) une séquence de nucléotides dite séquence pol codant pour les polypeptides dérivés dont une transcriptase inverse et une intégrase (polypeptides POL) d'un rétrovirus du type du rétrovirus de Friend,
   c) des signaux régulateurs de transcription contrôlant l'expression des séquences gag et pol, ledit vecteur d'expression pour la transcomplémentation étant dépourvu du signal d'encapsidation, et,
- d'autre part, un deuxième vecteur pour la transcomplémentation en protéines env comprenant une séquence de nucléotides codant pour les polypeptides d'enveloppe et des éléments de régulation de l'expression de cette séquence.

Dans le cadre de l'invention, on appelle un "virus du type de Friend", un virus dont les séquences codantes gag, pol ou env présentent suffisamment d'homologie avec celles du virus de Friend lorsqu'elles sont alignées avec elles, pour réaliser l'encapsidation d'ARN rétroviral telle que décrite dans la présente demande.

selon un premier mode de réalisation de l'invention, la séquence de nucléotides codant pour un ou les polypeptide(s) d'enveloppe est dérivée d'un rétrovirus de la famille des spumavirus. Il s'agit par exemple d'une séquence dérivée d'un rétrovirus HSRV.

Selon un autre mode de réalisation de l'invention, la séquence de nucléotides codant pour les polypeptides d'enveloppe est dérivée de la séquence 4070A du virus Mo-MuLV.

Les différentes lignées sont fabriquées par transfections successives sur le principe des lignées de troisième génération ; c'est-à-dire, avec fragmentation en deux parties voire en trois parties ou plus des séquences codant pour les protéines GAG, POL et ENV de complémentation et de leurs éléments de régulation. Cette construction limite les probabilités de recombinaisons génétiques susceptibles de générer des particules virales compétentes pour la réplication.

Selon un mode de réalisation de l'invention, les cellules d'encapsidation selon l'invention sont en d'autres termes caractérisées en ce qu'elles comprennent :
a) une séquence de nucléotides dite séquence gag codant pour les polypeptides dérivés d'une nucléoprotéine (polypeptides GAG) d'un rétrovirus du type du rétrovirus de Friend,
b) une séquence de nucléotides dite séquence pol codant pour les polypeptides dérivés dont une transcriptase inverse et une intégrase (polypeptides POL) d'un rétrovirus du type du rétrovirus de Friend,
c) des signaux régulateurs de transcription contrôlant l'expression des séquences gag et pol, et
d) une séquence de nucléotides dite séquence env, codant pour les polypeptides dérivés de l'enveloppe (polypeptides ENV) d'un rétrovirus de la famille des spumavirus, les polypeptides ENV permettant l'encapsidation d'ARN rétroviraux,
e) des signaux régulateurs de transcription contrôlant l'expression de la séquence env.

Les cellules d'encapsidation décrites ci-dessus sont destinées en outre à être transfectées par un vecteur rétroviral portant une séquence transgénique que l'on souhaite voir exprimer dans une cellule cible ultérieurement infectée par le surnageant des cellules d'encapsidation.

Selon un autre mode de réalisation, l'invention a pour objet une cellule d'encapsidation caractérisée en ce qu'elle comprend
a) une séquence de nucléotides dite séquence gag codant pour les polypeptides dérivés d'une nucléoprotéine (polypeptides GAG) d'un rétrovirus du type du rétrovirus de Friend,
b) une séquence de nucléotides dite séquence pol codant pour les polypeptides dérivés dont une transcriptase inverse et une intégrase (polypeptides POL) d'un rétrovirus du type du rétrovirus de Friend,
c) des signaux régulateurs de transcription contrôlant l'expression des séquences gag et pol, et
d) une séquence de nucléotides dite séquence env codant pour les polypeptides dérivés d'une enveloppe amphotrope, par exemple de l'enveloppe 4070A du virus MoMuLV,
e) des signaux régulateurs de transcription contrôlant l'expression de la séquence env.

Les cellules d'encapsidation de l'invention ont l'avantage d'être dépourvues de virus helper et dépourvues de virus endogènes, offrant ainsi des conditions de sécurité améliorées.

Les polypeptides GAG, POL, ENV dont il est question dans la demande correspondent soit à la totalité des polypeptides et/ou glycoprotéines exprimés par les séquences de nucléotides virales gag, pol, env, soit à une partie de ces polypeptides et/ou glycoprotéines ou à des formes modifiées notamment tronquées, si elles permettent de réaliser la transcomplémentation recherchée, en vue de l'infection des cellules cibles.

Une cellule particulièrement intéressante selon l'invention est caractérisée en ce qu'il s'agit de la cellule DOGOM 1 déposée à la CNCM sous le numéro I-1496, recombinée d'une part avec les séquences nucléotidiques gag et pol de la souche FB29 du rétrovirus de Friend, sous le contrôle des signaux de transcription contenus dans la séquence LTR de la souche FB29 du rétrovirus de Friend et recombinée d'autre part avec la séquence de nucléotides env d'un rétrovirus du type spumavirus sous le contrôle d'un promoteur inductible, par exemple le promoteur RARβ.

Le contrôle de la séquence codante de l'enveloppe de spumavirus par un promoteur inductible doit permettre de prévenir un effet lytique éventuel de l'expression de séquences de spumavirus, sur les cellules transfectées.

Une autre cellule recombinante transcomplémentante intéressante dans le cadre de l'invention, est caractérisée en ce que les signaux de transcription contenus dans la séquence LTR de la souche FB29 du rétrovirus de Friend contrôlant l'expression des séquences gag et pol, sont remplacés par un promoteur non viral suivi par la séquence de polyadénylation du virus SV40 ou par une autre séquence de polyadénylation.

Les séquences gag et pol peuvent ainsi être par exemple placées sous le contrôle du promoteur inductible RAR-β.

Dans un autre mode de réalisation, l'invention a pour objet des cellules recombinantes transcomplémentantes pour l'encapsidation d'ARN rétroviral, caractérisées en ce qu'elles comprennent les séquences gag et pol dont question ci-dessus, sous le contrôle d'un promoteur inductible ou conditionnel et une séquence env, par exemple amphotrope, par exemple dérivée de la séquence env 4070A du virus de Moloney également sous le contrôle d'un promoteur inductible ou conditionnel.

Dans un mode de réalisation particulier de l'invention, la cellule transcomplémentante est caractérisée en ce que la séquence de nucléotides du vecteur d'expression pour la transcomplémentation, codant pour les polypeptides d'enveloppe est modifiée, par exemple par remplacement de nucléotides par un enchainement de nucléotides codant pour une séquence d'un polypeptide ou d'une glycoprotéine reconnue spécifiquement par un type cellulaire défini, ou par l'addition d'un tel enchainement.

Les cellules recombinantes ainsi obtenues sont en outre transfectées par un vecteur rétroviral pour l'expression et/ou le transfert et/ou l'intégration au sein du génome d'une cellule cible, d'une séquence de nucléotides choisie ("séquence transgénique"), ce vecteur rétroviral étant avantageusement construit à partir de la souche FB29 du rétrovirus de Friend.

Ainsi, le vecteur rétroviral peut être construit en plaçant sous le contrôle de la séquence LTR, la séquence transgénique à exprimer, les séquences codant pour les gènes gag, env, pol étant délétées au moins en partie et la séquence d'encapsidation du rétrovirus étant présente au sein du vecteur.

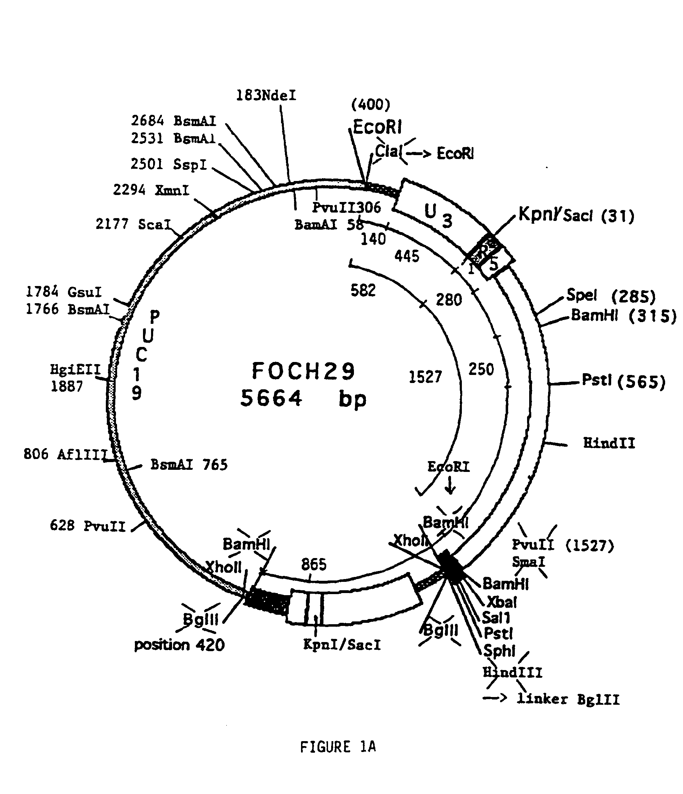

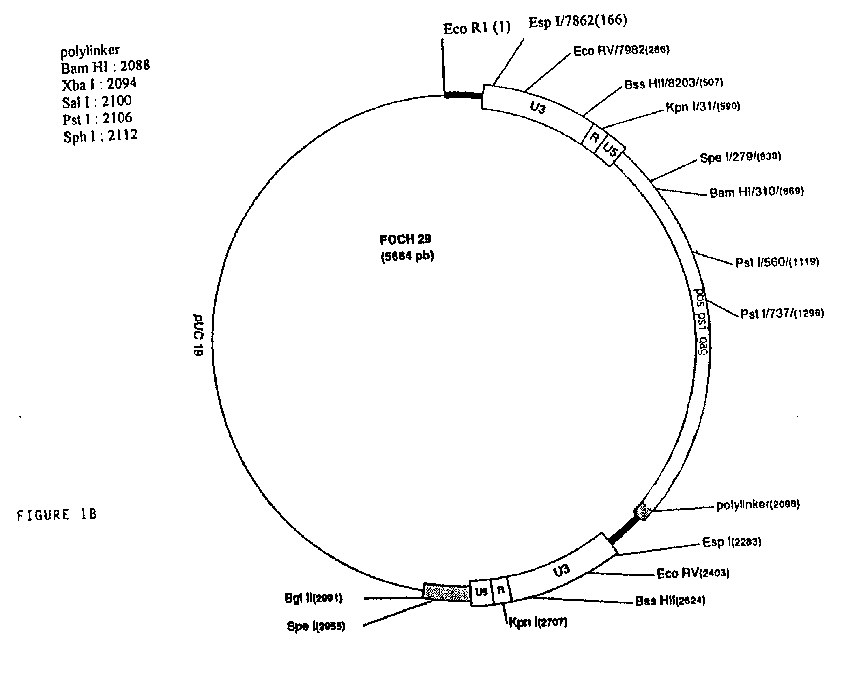

Un vecteur rétroviral ainsi utilisable pour transfecter les cellules transcomplémentaires dont question ci-dessus, est le vecteur pFOCH29 représenté à la figure 1 et déposé à la CNCM sous le n° I-1326, le 30 Juin 1993. La séquence transgénique à exprimer dans les cellules cibles est incorporée dans le vecteur pFOCH29 en un site non essentiel pour sa réplication.

D'autres vecteurs rétroviraux peuvent être utilisés. En particulier, on aura recours à des vecteurs construits à partir du vecteur pFOCH29 mais le cas échéant délétés au moins en partie au niveau des séquences LTR et par exemple au niveau du domaine U3 de la séquence LTR.

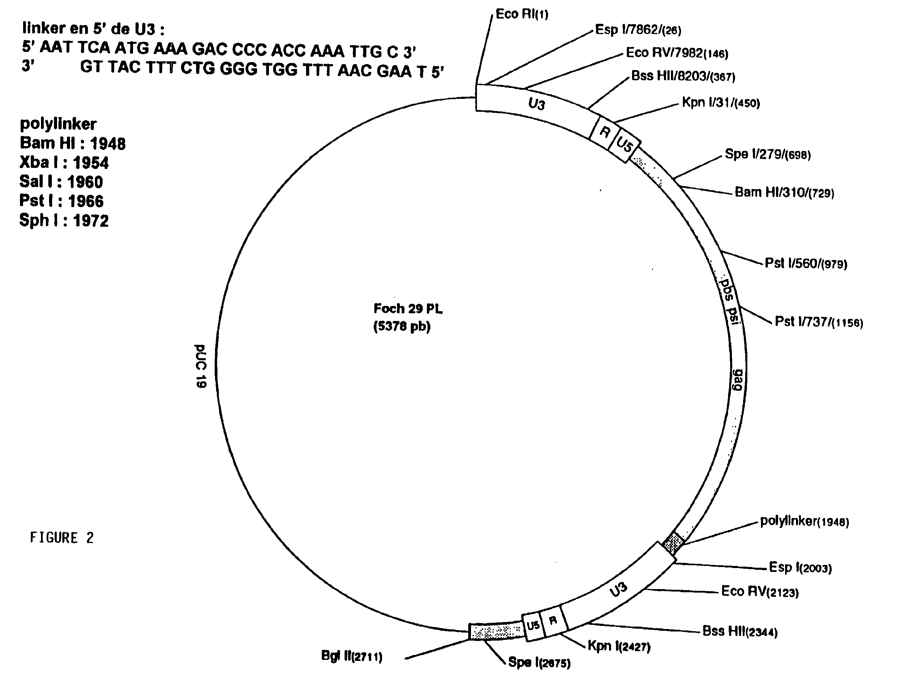

Un autre vecteur est le vecteur pFOCH29 PL représenté à la figure 2.

Dans le cadre de l'invention, tout vecteur rétroviral défectif pseudotypable par les lignées de complémentation définies ci-dessus, est susceptible d'être utilisé pour le transfert de transgènes.

Une séquence de nucléotides transgénique est une séquence de nucléotides qui n'est pas naturellement contenue dans le matériel génétique constituant le vecteur, et en particulier dans les séquences nécessaires au contrôle de l'expression du clonage ou du transfert. Il peut s'agir d'une séquence naturelle ou synthétique, notamment d'une séquence hybride.

Par l'expression "transfert d'une séquence de nucléotides transgénique", on entend l'incorporation d'une séquence portée par le vecteur, dans le génome ou satellite de celui-ci, d'une cellule cible transformée par ce vecteur. Un tel transfert peut être le résultat d'une recombinaison, en particulier d'une recombinaison homologue.

Le vecteur rétroviral peut donc permettre l'expression définitive dans le génome d'une cellule cible, d'une séquence de nucléotides exogène choisie du fait de sa propriété d'intégration au génome des cellules cibles.

Les vecteurs défectifs définis précédemment portant la séquence transgénique ou les plasmides-vecteurs d'expression pour les séquences gag et pol d'une part et env d'autre part pour la séquence de complémentation pour l'encapsidation, sont de préférence introduits par exemple par transfection ou électroporation, dans la lignée d'encapsidation. Cette transfection permet la constitution de particules virales, propres à la réalisation de recombinaisons par transduction dans des cellules cibles en vue du clonage, du transfert ou de l'expression de la séquence transgénique contenue dans le vecteur.

Les séquences d'intérêt thérapeutique susceptibles d'être introduites dans les cellules cibles grâce aux vecteurs et aux lignées d'encapsidation de l'invention sont par exemple des séquences correspondant à l'équivalent normal d'un gène non fonctionnel dans le cas d'une pathologie donnée, ou encore une séquence antisens ou un mutant négatif dominant d'un gène donné ou une séquence codant pour un inhibiteur fonctionnel d'un gène, ou l'utilisation d'un gène marqueur ou d'un gène régulateur ou pour une séquence de régulation d'un gène donné ou pour un gène susceptible d'ajouter une fonction nouvelle à la cellule cible.

L'invention offre ainsi des moyens, appropriés pour la thérapie génique du cancer par application des techniques de correction génique ou d'amélioration des stratégies de destruction des cellules tumorales. Selon la première technique, on effectuer une correction des mutations constitutionnelles dans le cas de la prédisposition héréditaire au cancer, des anomalies de la transduction du signal, comme les voies médiées par l'oncogène ras et ses homologues, des anomalies activatrices d'oncogènes, des anomalies inhibitrices de gènes suppresseurs de tumeurs, des anomalies favorisant l'instabilité génétique, des anomalies portant sur la réparation de l'ADN.

Selon la deuxième technique, l'invention peut être mise en oeuvre pour activer des prodrogues comme dans le cas du gène thymidine kinase du virus Herpès transformant le Ganciclovir ou l'Acyclovir en drogues cytotoxiques, ou le gène de Cytosine déaminase transformant un précurseur du 5-fluorouracile en drogue active, ou pour induire ou stimuler le système immunitaire par manipulation des cellules tumorales, avec par exemple des gènes de cytokines, par manipulation des cellules présentatrices de l'antigène ou de leurs précurseurs (souches hématopoiétiques) ou par manipulation des cellules effectrices de l'immunité, cellules T, cellules B, LAK, TILs.

S'agissant de la correction de maladies génétiques, d'anémies, l'invention est par exemple applicable à la correction d'erreurs innées du métabolisme, de maladies de l'hémoglobine, comme les thalassémies ou la drépanocytose, de maladies de l'hémostase et de la coagulation, de maladies héréditaires de la démyélinisation ou de myopathies.

L'invention peut aussi être mise en oeuvre dans la thérapie ou la profilaxie des maladies infectieuses du SIDA ; des pathologies cardiovasculaires ; pulmonaires ; pathologies cutanées ; hépato-digestives ; neuromusculaires ; des pathologies du système nerveux central, du pleuro-péritoine, etc.

Selon un autre mode de réalisation de l'invention, la séquence de nucléotides transgénique code pour un antigène ou un déterminant antigénique ou un antiidiotype.

Selon un autre mode de réalisation de l'invention, la séquence transgénique code pour un anticorps, en particulier un anticorps simple chaîne incluant notamment les séquences variables, responsables de la reconnaissance de l'antigène.

Un vecteur contenant un tel déterminant antigénique pourra être utilisé à titre de vaccin permanent ou transitoire ou le cas échéant dans le cadre d'un protocole thérapeutique, par exemple pour susciter une réponse immunitaire.

Les moyens de l'invention sont également appropriés pour vacciner des patients contre des agents pathologiques, et ce de façon permanente ou transitoire.

Le transfert peut être réalisé par transduction dans des cellules, tissus, organes ou organismes.

Les cibles cellulaires pour la mise en oeuvre de l'invention sont par exemple les fibroblastes, cellules endothéliales, mésothéliales et mésenchymateuses, cellules cutanées dont kératinocytes, cellules hépatiques et hépatocytes, cellules musculaires, cellules accessoires du système nerveux central, cellules gliales, oligodendrocytes, astrocytes etc... ; les cellules épithéliales dont l'urothélium, l'épithélium glandulaire dont la glande mammaire, l'épithélium pulmonaire, intestinal (etc...) ainsi que les lignées cellulaires quelle que soit leur origine.
Figure 1 : A. Carte de restriction du vecteur FOCH29.
   Côtes Seq "FB29"
      Cla --> U3 140 (7702-7845)
      U3 --> R 410 (7845-8255)
      R --> CBS 145 (0-145)
      PvuII --> BamHI 208
      PvuII --> PvuII 1098
      PvuIIMT --> PvuII 1669
      BsmAI --> 55/150/765/1766/2531/2684
   B: Carte de restriction de FOCH 29 dans laquelle les sites sont indiqués avec la numérotation de la séquence FB 29 de la figure 1A et avec la numérotation propre à la construction ainsi réalisée (numéros entre parenthèses).
Figure 2 : carte de restriction du vecteur rétroviral FOCH29.
Figure 3 :A, B: cartes de restriction du plasmide-vecteur contenant la construction LTR-FB29 Del Psi-gag/polFB29.
Figure 4 : cartes de restriction du plasmide-vecteur contenant la construction RAR-β-SD-Del Psi gag/polFB29.
Figure 5 : construction du plasmide vecteur "Pure LTR" contenant l'enveloppe amphotrope de l'enveloppe 4070A .
Figure 6 : construction du plasmide vecteur "Pure LTR" contenant l'enveloppe de spumavirus.
Figure 7 : constructions du plasmide vecteur RAR-β contenant l'enveloppe amphotrope 4070A.
Figure 8 : constructions du plasmide vecteur RAR-β contenant l'enveloppe de spumavirus.

### Exemples

### A) PREPARATION DU VECTEUR RETROVIRAL pFOCH29.

### MATERIEL ET METHODES

### 1- Source du matériel génomique viral

L'ADN génomique du provirus a été cloné dans pBR322 (Sitbon et al., 1986). Après remplacement du site ClaI en 7702 de la séquence du virus par un site EcoRI, le fragment EcoRI- PvuII de 2110 paires de bases (pb) contenant l'intégralité du Long Terminal Repeat viral (LTR) a été sous-cloné aux sites EcoRI et SmaI du polylinker (adaptateur multiple) de pUC19.

### 2- Construction du vecteur rétro-viral FOCH29

Le site HindIII du polylinker de pUC19 contenant le fragment EcoRI-PvuII a été remplacé par un site BglII, après ouverture par HindIII, remplissage par le fragment long de l'ADN polymèrase d'E.Coli (fragment Klenow) et ligation avec un adaptateur BglII ne recréant pas le site HindIII ; le site BglII a été introduit pour recevoir un fragment BamHI-BamHI de 865pb contenant un second exemplaire du LTR natif du virus de Friend destiné à constituer le LTR d'aval (ou 3'LTR). Ce fragment BamHI-BamHI a été isolé en remplaçant le site EcoRI de pUC19 par un site BamHI d'amont au moyen d'un adaptateur (linker) ne recréant pas le site EcoRI après remplissage des extrêmités par le fragment Klenow de l'ADN polymèrase ; le site BamHI d'aval est endogène à la séquence virale.

Ce fragment a donc été introduit par ligation avec l'armature de base (pUC19) dont l'ouverture par BglII permettait de ménager des extrémités cohésives avec les extrémités générées par BamHI. Le plasmide résultant est appelé pFOCH29.

### 3- Introduction d'un gène marqueur

Le fragment d'ADNc BglII-BamHI (1500 pb) du gène de résistance à la Néomycine dérivé du rétrotransposon Tn5 (NéoR) a été introduit entre les deux LTRs viraux après ligation des trois fragments : pUC19-5'LTR BglII, NéoR BglII-BamHI et 3'LTR BamHI-BamHI. Le plasmide résultant est appelé pFOCH29-Néo.

### 4- Transfection de lignées d'encapsidation Psi-CRIP et infection de fibroblastes

Le plasmide pFOCH29-Néo a été introduit dans la lignée d'encasidation amphotrope psi-CRIP décrite par Danos et al. (1988) par transfection utilisant la précipitation au phosphate de calcium selon la procédure standard, sans ADN carrier ; 10 microgrammes de plasmide ont été déposés sur une boîte de culture de 35mm de diamètre où 5.10⁴ cellules ont été plantées la veille.

Les cellules psi-Crip ont été cultivées en milieu de Dulbecco modifié Eagle (DMEM, Gibco-BRL) additionné de 10% de sérum de veau nouveau-né (HyClone). Deux jours après la transfection les cellules ont été trypsinées, diluées au 1/20 ème et mises sous sélection, en présence de Généticine à la concentration finale de 1 milligramme (mg) par Millilitre (ml) de milieu de culture. Les colonies apparues après 12 jours ont été prélevées et réimplantées sur des boîtes de culture de 24 puits, à raison d'un clone par puits.

Le surnageant de culture de cellules d'un puits parvenu à confluence a été prélevé, filtré sur 0,45 µm pour éliminer les cellules en suspension et utilisé pour infecter des fibroblastes murins (NIH3T3) plantés de façon identique sur des boîtes de culture de 24 puits, en présence de polybrène à la concentration de 8 *µ*g/ml. Les NIH3T3 ont été cultivées en DMEM additionné de 10% de sérum de veau foetal (SVF). L'intégration virale a été analysée par PCR sur un lysat de NIH3T3 parvenues à confluence.

### 5- Réaction en chaîne de la Polymérase (Polymerase Chain Reaction ou PCR)

Le surnageant du lysat des NIH3T3 confluentes sur un puits de boîte de culture de 24 puits a été recueilli dans 100 *µ*l dont 10 ul ont été utilisés dans la réaction de PCR, qui est pratiquée dans le tampon suivant : tampon de PCR 10X standard incluant 25mM de MgCl₂ (Perkin-Elmer/Roche MS) ; 100 nanogrammes (ng) de chaque amorce ; 2ul de dNTPs 10mM (mélange équimolaire de chaque dNTP à la concentration initiale de 10 mM, soit 2,5mM chacun) ; 2 unités de Taq Polymèrase clonée (Perkin-Elmer/Roche MS) pour 40 cycles, une seule unité pour 25 cycles ; pour un volume final de 50*µ*l.

Deux couples d'amorces ont été utilisés.

Les séquences des oligonucléotides utilisés sont : 1°) - pour le premier couple : 5'CTGCTGACGGGAGAAGAAAAAC-3' / 5'CCCGCTCAGAAGAACTCGTC-3' ; 2°) - pour le second couple : 5'GACGAGTTCTTCTGAGCGGG-3' / 5'GATCTGAACTTCTCTATTCTTG-3'

La taille des séquences amplifiées est pour le premier couple, Fin-gag/deux tiers proximaux gène NéoR : 900 pb ; et pour le second couple, tiers distal gène NéoR/ moitié proximale du LTR 3': 610 pb.

Dénaturation 5 min à 94°C ; 40 cycles sur GeneAmpPCR 9600 avec dénaturation 30" à 94°C ; annealing 15" à 55°C et élongation 30" à 72°C ; suivis d'une étape terminale d'élongation de 10 min.

Les échantillons (15ul sur 50ul) ont été déposés sur gel d'agarose à 1,2% (Seakem, FMC) et ont été soumis à une électrophorèse horizontale de 45 min à 80 volts ; la détection du signal reposant sur l'analyse de l'intensité de fluorescence en bromure d'éthidium (BET).

### 6- Détermination des titres infectieux

Chacun des clones ayant été testé pour sa capacité à infecter des NIH3T3 a été amplifié et éventuellement congelé dans l'attente de l'analyse par PCR de l'efficacité d'infection.

Après PCR deux clones principaux ont été retenus et amplifiés afin d'infecter des NIH3T3 selon une procédure standard.

1ml de surnageant de culture de 16 heures sur une boîte de 35mm de diamètre de chaque clone producteur a été prélevé à confluence, filtré sur 0,45um afin d'éliminer les cellules productrices en suspension. Le surnageant a été mis en contact avec les cellules NIH3T3 à 50% de confluence sur une boîte de culture de même diamètre (35mm), en présence de polybrène à la concentration de 8ug/ml de milieu. Les cellules ont été incubées pendant 2H30 environ, à 37°C ; une agitation a été pratiquée chaque demi-heure. Trois volumes de milieu frais ont été ajoutés après 2H30.

### TITRES INFECTIEUX VIRAUX

Des dilutions successives du surnageant primaire ont été utilisées pour infecter des cellules NIH3T3 ; surnageant pur, dilué au 1/10ème, au 1/1000ème et au 1/100 000ème. Deux jours après l'infection, les cellules ont été trypsinées passées au 1/20ème environ sur trois boîtes de culture de 100 mm de diamètre et mises sous sélection par addition de Généticine (lmg/ml) au surnageant.

Cette procédure expérimentale a été rendue plus stringente dans la mesure où : d'une part, la drogue est ajoutée très vite après l'infection ; et d'autre part, la mise en sélection directe sans trypsiner les cellules ne permet pas de multiplier artificiellement le nombre de colonies résultantes, les cellules filles issues d'une cellule infectée restant groupées et ne formant qu'une colonie unique in situ. A l'inverse, lorsque les cellules sont trypsinées, les cellules filles essaiment dans la boîte de culture réceptrice et forment des colonies artificiellement indépendantes qui, si elles sont dénombrées, multiplient artificiellement le titre.

### SOUTHERN BLOT

Deux jours après l'infection par du surnageant pur, les NIH3T3 ont été trypsinées, passées au 1/20ème environ sur trois boîtes de culture de 100 mm de diamètre, dont l'une est mise sous sélection par la généticine.

A confluence, l'ADN génomique des cellules infectées par chacun des deux clones après ou sans sélection, est extrait puis quantifié.

L'ADN a été digéré par deux enzymes de restriction, PstI et KpnI afin de réaliser un Southern blot. Après contrôle de la qualité de la digestion et homogénéïsation de la quantité d'ADN déposée dans chaque puits, le transfert a été réalisé sur une membrane de Nylon Hybond N (Amersham). L'hybridation a été conduite avec une sonde incluant la totalité des séquences du LTR viral encadrées par 100 bases en amont et 100 bases en aval. La sonde a été marquée par extension d'amorce (Feinberg et Vogelstein, 1983, 1984) avec du dCTP marqué à l'alpha-32-P, à une activité spécifique de 5.10⁸ cpm/µg.

L'hybridation a été pratiquée dans un milieu constitué de : 50% formamide désionisée ; 5X SSEP ; 1X denhardt's : 5% dextran sulfate et 0,2mg/ml d'ADN de saumon soniqué, pendant 20 heures à 42°C. De brefs rinçages ont été pratiqués dans une solution peu stringente 2X SSEP/0,1% SDS, 5min à température ambiante et 10 min à 65°C ; suivis d'une exposition de 3 jours sur films Kodak-XAR-5 à -80°C avec écrans amplificateurs Li-Plus (Dupont-NEN).

### 7- Recherche de production de virus Helper

Cette recherche a été effectuée par test de mobilisation sur lignées 3T3BAG (Danos et al, 1988 ; Danos, 1991).

Les cellules 3T3BAG ont dans un premier temps été infectées par le surnageant pur de lignées d'encapsidation infectées. Plusieurs cycles successifs d'infection de 3T3BAG vierges avec le surnageant des 3T3BAG précédemment infectées ont été pratiqués pour sensibiliser le test.

### RESULTATS

### 1- Construction du vecteur rétro-viral FOCH29

La souche virale FB29 du virus de la leucémie murine de Friend a été isolée (Mathieu-Mahul et al., 1982) et l'ADN génomique du provirus intégré a été cloné dans pBR322 (Sitbon et al;, 1986). Cet ADN génomique a été entièrement séquencé (Perryman et al., 1991). Le fragment génomique ClaI-PvUII de 2120 pb a été sous cloné dans pBR322. Ce fragment comporte les derniers nucléotides de la séquence codant pour la p15E de l'enveloppe virale, l'intégralité du Long Terminal Repeat (ou LTR) et les 3/5 èmes des séquences gag. Il constitue la matrice de l'architecture du vecteur.

Après remplacement du site ClaI par un site EcoRI, le fragment EcoRI-PvuII a été sous-cloné respectivement en EcoRI-SmaI de l'adapteur multiple de pUC19. Ce clone a, d'une part, été maintenu intact pour former l'architecture de base du vecteur incluant : le LTR d'amont ou LTR 5', le site de fixation de l'initiateur de la transcription virale (Primer Binding Site ou PBS), la séquence d'encapsidation, les séquences gag et le segment de l'adaptateur multiple (polylinker) de pUC19 ménagé par la digestion EcoRI/SmaI ; destiné à l'insertion de gènes d'intérêt.

D'autre part, un fragment BamHI-BamHI a été dérivé, en remplaçant en amont, le site EcoRI par un site BamHI ; et en tirant profit, en aval, du site BamHI endogène du virus, situé immédiatement en aval du site donneur d'épissage. Ce fragment a été introduit dans l'armature de base, dont le site HindIII du polylinker avait été au préalable remplacé par un site BglII, générant des extrémités cohésives avec celles générées par l'enzyme BamHI.

Le gène marqueur dérivé du Rétrotransposon Tn5, conférant une résistance à la Néomycine (NéoR), a été introduit entre les deux LTRs. Après transformation sur une souche de bactéries hypercompétentes de phénotype recombinase négatif profond, afin de prévenir des remaniements éventuels des séquences en présence, les transformants de la configuration attendue ont été sélectionnés sur la base d'une carte de restriction étendue explorant la totalité de la construction. L'un d'entre eux, baptisé pFOCH29 a ensuite été amplifié et purifié afin de disposer d'une source de matériel adéquat, destiné à la transfection de lignées auxiliaires productrices de particules virales.

### 2- Obtention de clones producteurs de virus défectif

Transfection de lignées d'encapsidation psi-CRIP : Le plasmide pFOCH29-Néo a été introduit dans la lignée d'encasidation amphotrope psi-CRIP décrite par Danos et al. (1988) par transfection utilisant la précipitation au phosphate de calcium selon la procédure standard, sans ADN carrier.

Après la mise sous sélection par la Généticine, 40 parmi les colonies apparues ont été prélevées et le surnageant de culture utilisé pour infecter des fibroblastes murins (NIH3T3) Le processus primaire de sélection d'une série de clones les plus hautement producteurs de particules virales défectives encapsidées a reposé sur l'utilisation de la technique d'amplification génique par réaction en chaîne de la polymérase. L'intégration virale est analysée par PCR sur un lysat de NIH3T3 parvenues à confluence.

Deux couples distincts d'amorces de PCR ont été utilisés : 1° un premier couple amplifiant le segment terminal des séquences gag incluses dans la construction et les deux tiers proximaux du gène de résistance à la néomycine ; 2° un second couple d'amorces amplifiant le tiers distal du gène de résistance à la Néomycine et la moitié du LTR d'aval (3').

Quatre clones ont été retenus sur la base d'un signal de PCR plus intense que les 36 autres ; la répétition de la procédure a permis de confirmer les données initiales indiquant que pour deux clones le signal se détachait de façon nettement plus intense. Ces deux clones ont été amplifiés et le surnageant de culture des cellules productrices a ensuite été utilisé pour infecter des NIH3T3 à plus large échelle dans le but d'évaluer l'efficacité de la construction en termes quantitatifs.

### 3- Evaluation des clones producteurs

### PCR Quantitative

Une analyse par PCR semi-quantitative a été menée en utilisant le couple d'amorces amplifiant la région correspondant au tiers distal du gène NéoR jusqu'à la partie médiane du LTR d'aval (3'LTR). Pour chaque clone 1*µ*g et 3*µ*g d'ADN génomique extrait à partir de cellules NIH3T3 infectées par un surnageant pur après ou sans sélection par la Néomycine, ont été utilisés. Chaque test a été effectué en double (duplicate). Plusieurs dilutions du plasmide pFOCH29-Néo ont été testées en parallèle calculées de telle sorte qu'elles correspondent à 0.1, 0.5 et 1 copie du transgène pour l'équivalent de 1*µ*g d'ADN génomique soit respectivement 0,115 pg, 0,575 pget 1,15 pg pour un plasmide d'une taille de 7164 pb .

La PCR a été réalisée sur 24 cycles, ce qui correspond à une phase encore exponentielle de la réaction. La lecture a été réalisée par analyse densitomètrique informatisée (Cybertech) de la fluorescence au bromure d'éthidium.

Pour le premier clone, une différence significative a été observée entre l'intensité du signal obtenu à partir des cellules infectées sélectionnées et non sélectionnées ; plus franche sur les échantillons de 1*µ*g (70% du signal par rapport au sélectionné) que sur ceux de 3*µ*g pour lesquels le système de détection est saturé par l'intensité du signal.

Pour le deuxième clone, il n'existe aucune différence d'intensité du signal entre cellules sélectionnées et non-sélectionnées, ni pour les échantillons de 1µg, ni pour ceux de 3µg. Ceci suggérant qu'un pourcentage proche de 100% des cellules NIH3T3 ont pu être infectées par le surnageant pur de ce clone producteur. Le haut degré d'infectivité de ce clone était par ailleurs suspecté par l'observation d'une absence de mortalité cellulaire lors de la mise sous sélection par la Néomycine des NIH3T3 infectées avec le surnageant de culture.

### Southern Blot

L'ADN des cellules NIH3T3 infectées a été soumis à une hydrolyse avec deux enzymes de restriction : KpnI et Pst I. Selon les sondes utilisées, la taille des bandes attendue après intégration virale varie. Pour l'enzyme KpnI qui coupe à l'intérieur des LTRs et au milieu de l'adaptateur multiple de pUC19, une sonde LTR révèle un fragment de taille constante de 3610 pb et deux fragments de taille variable selon la proximité de sites KpnI génomiques endogènes à proximité du site d'intégration ; une sonde dérivée par PCR avec les amorces tiers distal NéoR/segment proximal LTR, un fragment de même taille (3610 pb) est attendu, et un seul des deux autres fragments variables d'une intégration à l'autre.

Pour l'enzyme PstI qui coupe deux fois dans la partie médiane de gag et une fois dans l'adaptateur multiple de pUC19, après intégration les fragments allumés par les deux sondes précédentes seront de taille variable ; une sonde générée par PCR à partir du second couple d'amorces allume un fragment constant de 790 bases.

Plusieurs dilutions du plasmide pFOCH29-Néo digéré par KpnI et PstI ont été adjointes sur le Southern blot ; ces dilutions correspondant respectivement à 0.1, 0.5 et 1.0 copies du plasmide pour 10 *µ*g d'ADN génomique.

En outre, l'ADN de cellules infectées non sélectionnées après l'infection et des mêmes cellules sélectionnées par la Néomycine a été systématiquement juxtaposé afin de quantifier l'infectivité de la construction ; les cellules ayant fait l'objet d'une sélection constituant un témoin d'infection de 100%.

### Titration : Titres infectieux viraux par dilutions virales

Des dilutions successives du surnageant primaire ont été utilisées pour infecter des NIH3T3 ; surnageant pur, dilué au 1/10ème, au 1/1000ème et au 1/100 000ème. Les cellules sont infectées par du surnageant viral, à raison de 0,5 ml sur un puits de 35 mm de diamètre : la drogue de sélection est ajoutée dès 20 heures après l'infection directement sur la boîte de culture, sans trypsiniser les cellules. Le titre infectieux retenu correspond au nombre de colonies observées pour la dernière dilution où des colonies apparaissent que multiplie l'inverse de cette dilution.

Pour le premier clone, le titre des cellules productrices initiales est de 2.10⁶ pfu/ml. Pour le deuxième clone, le titre est de 10⁶ pfu/ml

Les deux clones producteurs ont été d'une part congelés régulièrement afin d'en conserver des passages initiaux ; et d'autre part, maintenus en culture continue sur plusieurs mois. Les titrations successives (dilutions du surnageant viral) ont permis d'identifier une baisse progressive des titres. Pour le premier clone, le titre est passé de plus de 2.10⁶ à seulement 10¹ en l'espace de deux mois de culture continue ; cette chute drastique du titre s'est accompagnée d'une modification de la pousse des cellules en culture avec un aspect en foyer et un décollement facile. Pour le second clone, le titre est passé de plus de 10⁶ à 10⁵ en l'espace de deux mois de culture continue, pour diminuer jusqu'à 10³-10⁴ après trois mois et demi ; cette chute modérée du titre ne s'est accompagnée d'aucune modification de la morphologie ni de la pousse des cellules en culture.

### 4- Test activité helper sur 3T3 BAG

Cette recherche a été effectuée par test de mobilisation sur lignées 3T3BAG (Danos et al, 1988 ; Danos, 1991).

Les cellules 3T3BAG ont dans un premier temps été infectées par le surnageant pur de lignées d'encapsidation infectées. Plusieurs cycles successifs d'infection de 3T3BAG vierges avec le surnageant des 3T3BAG précédemment infectées ont été pratiqués pour sensibiliser le test, qui s'est révélé négatif. En outre, aucune colonie de cellules résistant à la Néomycine n'a pu être détectée après mise en contact de NIH3T3 vierges avec le surnageant de NIH3T3 infectées et sélectionnés par la Néomycine.

### 5) SITES D'INTEGRATION

Des cellules de primate humain et non-humain ont été utilisées pour identifier le nombre de sites d'intégration du virus après infection. Les cellules murines fournissent des indications de qualité très moyenne, dans la mesure où il existe sur ces cellules un bruit de fond significatif lié aux multiples intégrations de séquences rétrovirales ou rétrovirales-like.

A cette fin, des cellules VERO de singe ont été infectées avec plusieurs dilutions de surnageant viral. Pour la dilution 10⁻², des clones indépendants ont été obtenus ; chacun ayant été initié à partir d'un seul profil d'intégration virale. L'utilisation d'une enzyme de restriction coupant d'une part à l'intérieur de la construction virale et d'autre part, dans l'ADN génomique de la cellule hôte à des distances variables des sites d'intégration, a permis d'obtenir autant de fragments de restriction, de taille variable, qu'il y a eu d'évènements d'intégration. Ici, les enzymes XbaI ou SalI ont été utilisées.

### 6) STABILITE DES TITRES VIRAUX - MASTER BANK SYSTEM

Un stock homogène de cellules productrices de virus a été constitué et contrôlé extensivement pour l'absence de virus compétent pour la réplication par les méthodes suivantes :
- Test de Mobilisation sur cellules 3T3BAG
- Amplification sur NIH3T3
- Innoculation à des souris nouveau-né en intrapéritonéal,
afin d'étudier une éventuelle pathogénèse in vivo.

A partir de cette banque de cellules ("Master Cell Bank" MCB), une banque de cellules de travail (Working Cell Bank) a été constituée. Les titres viraux obtenus sont restés stables sur plusieurs mois et étaient de l'ordre de 20 x 10⁶ à 3 x 10⁷ cfu/ml (les dilutions au départ n'ayant pas été poussées de façon systématique que à 10⁻⁶. Des résultats ont aussi été obtenus, avec un test incluant une dilution à 10⁻⁷. Les titres sont remarquablement stables sur plusieurs mois à ce niveau d'intensité.

### B) CONSTRUCTIONS RETROVIRALES ADDITIONNELLES

1) - Une construction a été faite avec un LTR encore plus épuré en amont des LTR 3' en particulier ; Dénomination **FOCH29-PL** (pour FOCH29 Pure LTRs) représenté à la Figure 2.

Cette construction a permis d'évaluer le bénéfice en terme de stabilité génétique de l'excision des 140 bases, dont 104 de la fin de l'enveloppe, en amont des LTRs viraux.

La construction a été effectuée à partir du plasmide pUC19 incluant le fragment EcoRI-PvuII décrit plus haut : coupure enzymatique par l'enzyme de restriction EspI (ou IsoCelII) en position 7864 (soit +23 du LTR viral). A l'extrémité 5' les bases générées par une coupure EcoRI ont été artificiellement ajoutées sur un oligonucléotide de synthèse double brin complémentaire des 23 bases du LTR (140 bases dont 103 bases d'enveloppe). En 3' l'oligonucléotide est complémentaire des extrémités cohésives SpeI. Les séquences des oligonucléotides sont les suivantes : Oligo-SENS 5' - AAT TCA ATG AAA GAC CCC ACC AAA TTG C -3' ; Oligo-ANTISENS 5' - TAA GCA ATT CGG TGG GGT CTT TCA TTG -3'.

### C) CONSTRUCTIONS POUR LA COMPLEMENTATION DANS LES LIGNEES D'ENCAPSIDATION

### 1) Construction GAG-POL pour à la mise au point d'une lignée d'encapsidation originale.

Les étapes impliquant l'utilisation des séquences dérivées de la souche FB29 du virus de Friend sont décrites ci-dessous.

La construction gag/pol complémentant pour les protéines de nucléocapside et la transcriptase inverse est dérivée de la souche FB 29 du virus de Friend.

La construction de base a été réalisée à partir de la construction décrite dans les pages précédentes pFOCH29 où le LTR était laissé en place ou remplacé par les séquences du promoteur RAR-β.

Une délétion large des séquences d'encapsidation situées en amont des séquences codant pour les nucléoprotéines de capside gag (commençant en + 619) est pratiquée comme suit : coupure SpeI (ou IsocelII), site unique en + 280 ; et PstI en + 560-561 qui enlève 280 bases. Un adaptateur synthétique SpeI-PstI est synthétisé : 5' -CTAGTGCA-3' et apparié ("annealing") au plasmide, recoupé par HindIII. Une ligation est ensuite pratiquée avec le troisième fragment PstI-HindIII incluant la majeure partie des séquences GAG et POL.

Alternativement l'adaptateur synthétique SpeI-PstI a été remplacé par un simple linker PstI.

Puis, dans un deuxième temps (après transformation et sélection des recombinants positifs), le fragment PstI-PstI (561-737), incluant l'ATG du GAG a été cloné au site PstI dans sa position originale, afin de rétablir l'intégralité des séquences GAG ; l'orientation du clonage de ce petit fragment symétrique PstI-PstI est établie par une digestion enzymatique HaeII (position 720 ; un second site est situé en 4291, mais ne gène pas l'orientation) / AhaIII (site unique sur toute la séquence FB29, position 7864 du LTR) ou EspI (site unique sur toute la séquence FB29, position 7864 du LTR). Du fait de la méthode utilisée pour le clonage au site HindIII de pUC19 des séquences d'ADN correspondant à la totalité du génome de FB29, la fin des séquences POL a été récupérée comme suit : coupure initiale par HindIII (5060) et SnaI (= iso-Bst11071) (site unique en 6335) ; après purification, ce fragment a été recoupé par SmaI (6079), pour obtenir un fragment de 1019 bases, comportant un minimum de séquences d'enveloppe (244 bases). Ce fragment a été sous cloné en HindIII- HincII du polylinker de pUC18. Le signal de polyadénylation de SV40 a été juxtaposé en aval (excisé du plasmide pCRIPgag-2, Danos et Mulligan, 1988) de la construction.

Un fragment NheI en 5'- EcoRI en 3' est excisé d'un plasmide contenant le polyA de SV40, incluant le fragment HpaI-EcoRI (position 2666 à 1782) du signal de polyadénylation du promoteur précoce de SV40. Ce fragment est ensuite cloné [en XbaI (cohésif NheI- soit en digestion partielle ménagée car il existe un site XbaI à la fin de POL de Friend ; soit en étape préalable au clonage dans pUC18 de la fin de POL) ou en BLUNT ou après avoir introduit un linker SpeI (cohésif de la coupure NheI) au site KpnI du polylinker de pUC18] et en aval, cohésif EcoRI.

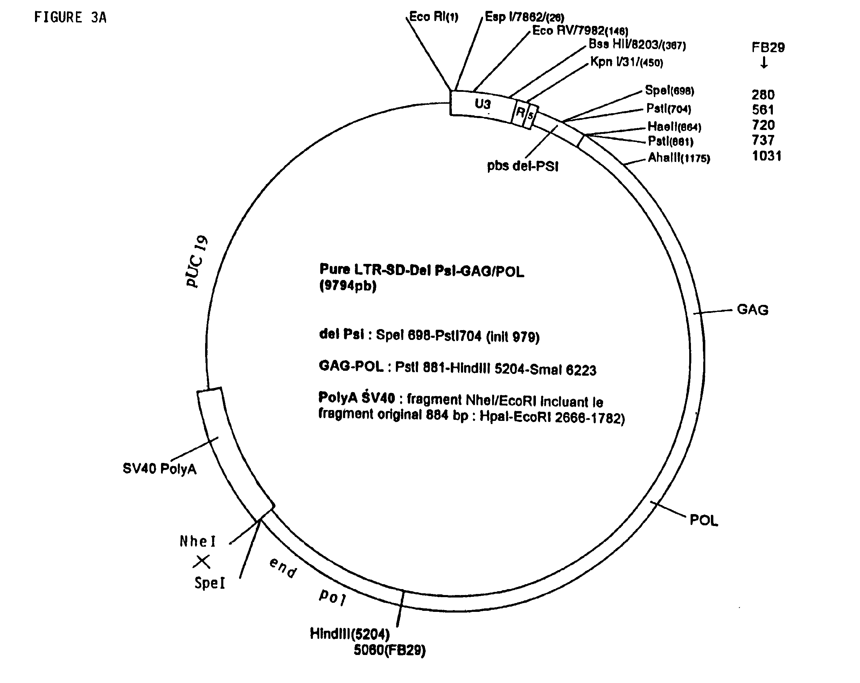

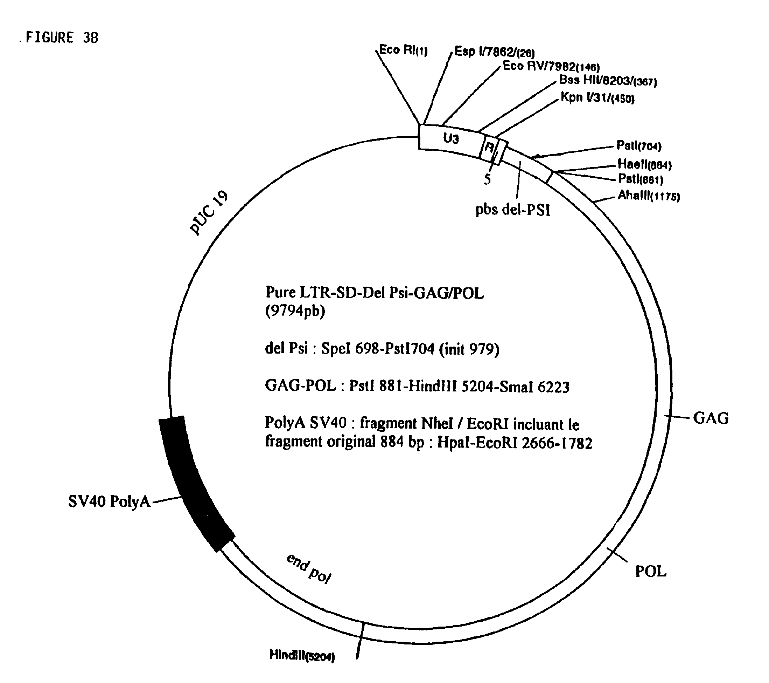

Les séquences POL et Poly-A ont été excisées en bloc de pUC18 par une digestion HindIII en 5' et EcoRI en 3' et une ligation a été pratiquée avec le plasmide pUC19/LTR épuré/del- Psi/GAG/2/3-POL, décrit ci-dessus (figure 3).

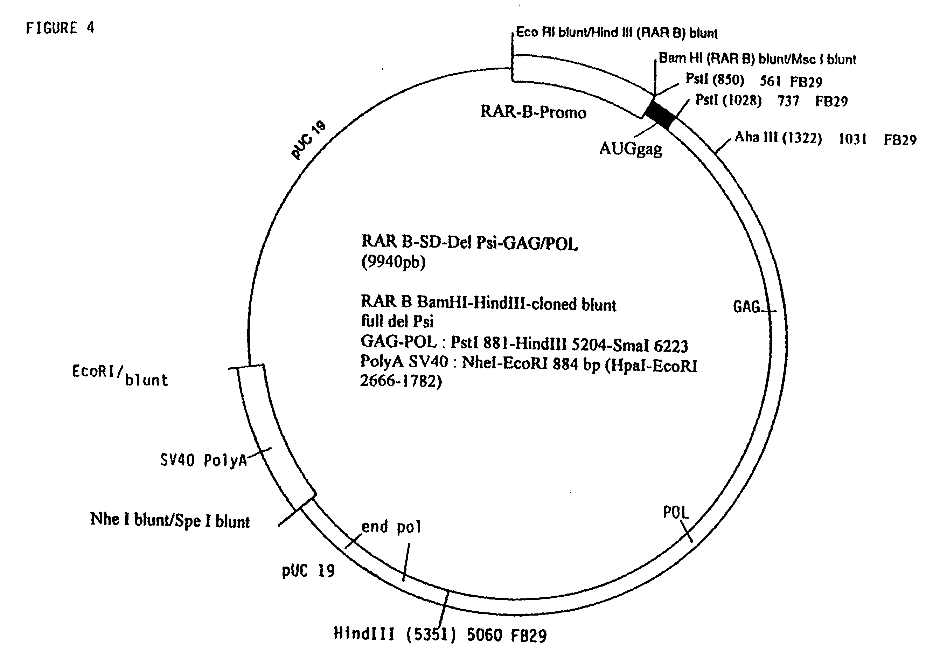

Une construction similaire peut être réalisée en remplaçant le LTR viral par un promoteur non viral tel que le promoteur RAR-β (figure 4A).

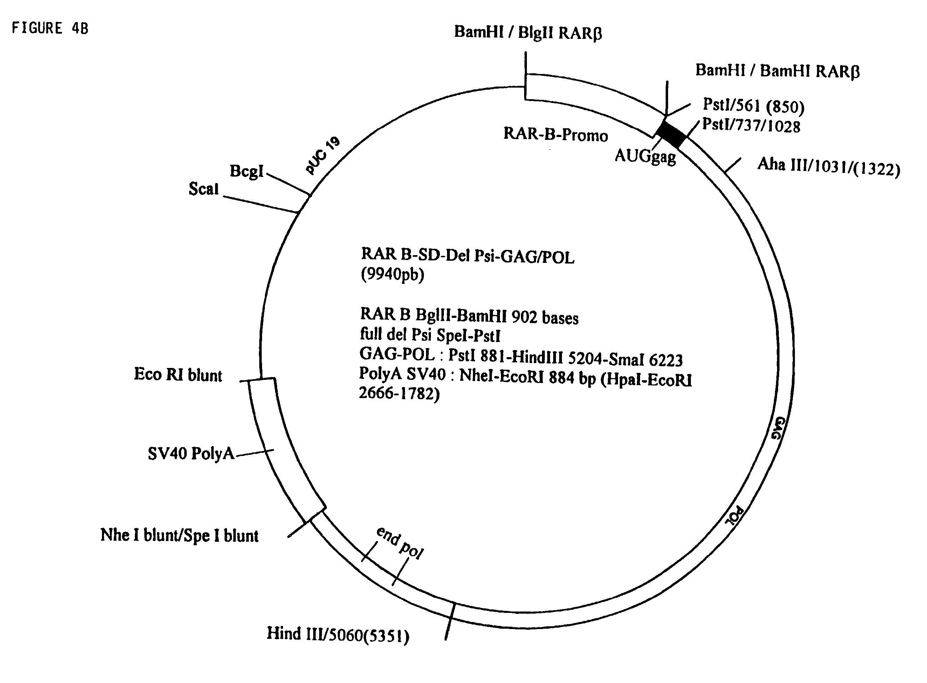

Dans ce cas, le fragment BglII-BamHI du promoteur RAR-β est cloné au site BamHI d'un plasmide pUC19 (fragment BglII-BamHI dont les deux extrémités sont cohésives BamHI). L'orientation est établie à partir du site EcoRI situé à 85 bases du site BamHI (extrémité 3') et dégageant deux fragments très asymétriques dans le promoteur RAR-β. Ce plasmide est ouvert en BcgI ou ScaI (5') et AflIII (3') afin de purifier dans un second temps le fragment BcgI ou ScaI/PstI (3') contenant le promoteur RAR-β (respectivement 1803 ou 1841). Sans la purification préalable avec Af1III (respectivement 2200 et 2250 bases), la séparation des fragments générés par la coupure PstI est impossible. Le fragment purifié est ensuite ligué au fragment PstI/ScaI ou BcgI excisé du plasmide LTR-GAG/POL, contenant les séquences GAG/POL débarrassées du LTR viral (7837 ou 7875 bases). La construction est achevée en restituant le fragment PstI-PstI de 176 bases du GAG après ouverture de la construction précédente par PstI et clonage orienté (déjà décrit plus haut) du petit fragment (figure 4B).

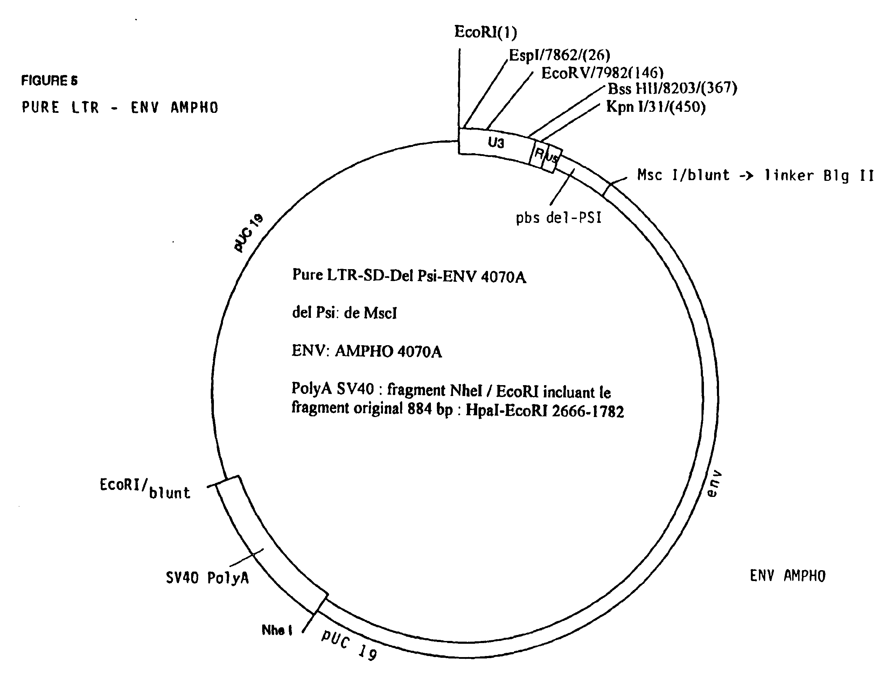

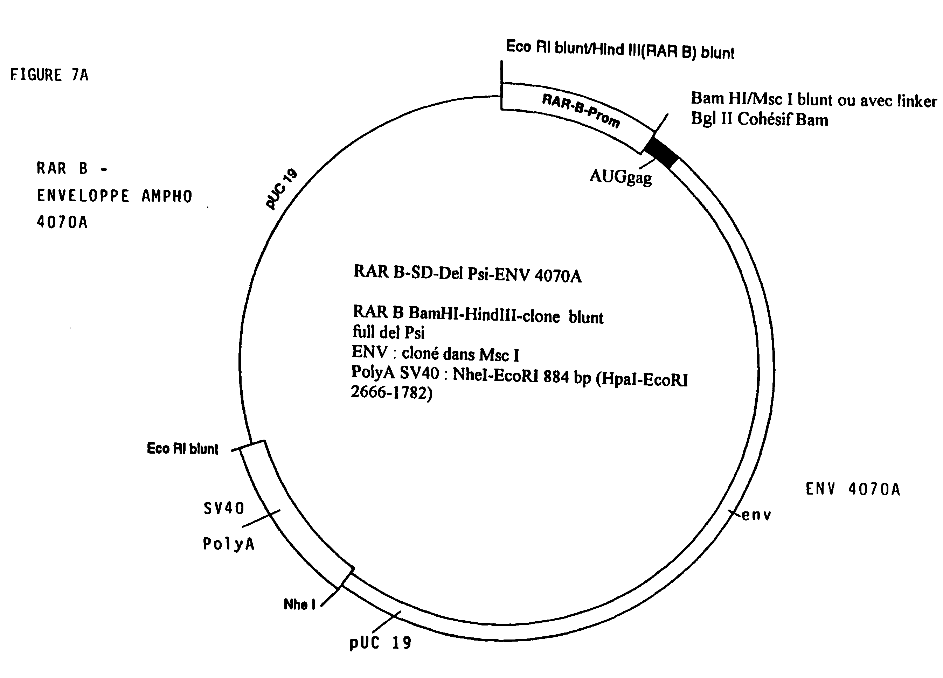

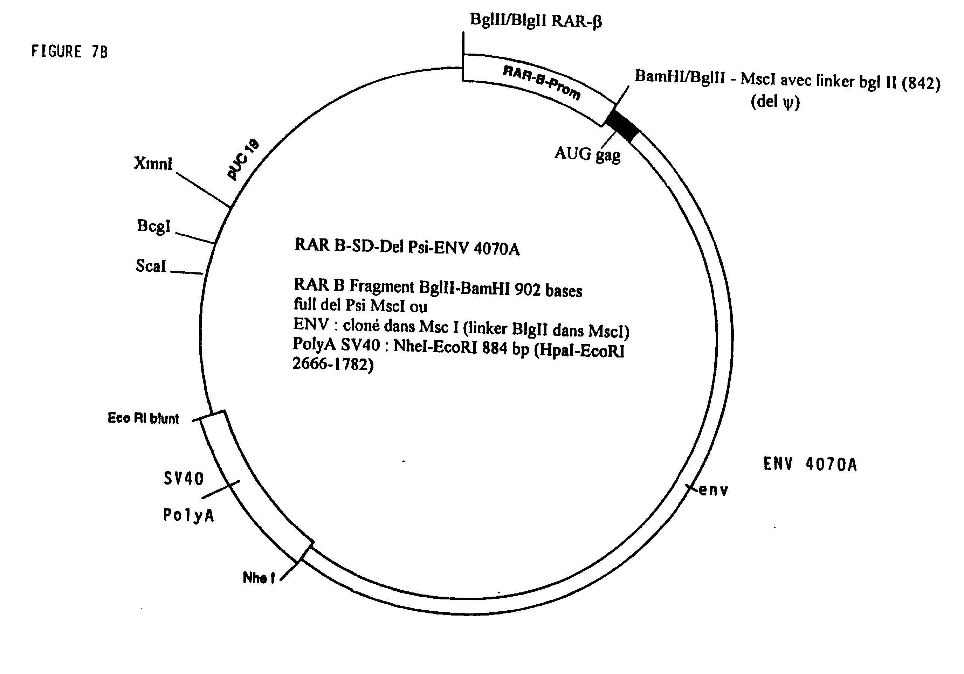

### 2) Construction ENV amphotrope (Figures 5 et 7)

Les séquences d'enveloppe amphotropes 4070A, dérivées d'une souche amphotrope du virus de la leucémie de Moloney (Mo-MuLV) (Chattopadhyay et al, 1981), similaires à celles utilisées dans la lignée psi-CRIP sont utilisées pour la complémentation en enveloppe. La transfection est réalisée en co-transfection avec un second gène de sélection : ou Histidinol ou Hygromycine Deux options :
a) Transcription sous 5'LTR viral avec site de polyadénylation d'aval de SV40 : un fragment BglII-EcoRI est excisé de la construction pCRIPAMgag-, et ligué à la construction pure LTR, après introduction d'un linker BglII en MscI de la séquence FB29, pour un clonage cohésif en 5' ; et XbaI blunt (vecteur)-EcoRI blunt (fin du signal PolyA de SV40) pour le clonage en 3'.
b) Transcription sous promoteur inductible par un traitement pharmacologique, en particulier le promoteur sus-décrit du récepteur béta à l'acide rétinoïque : les séquences d'enveloppe venant remplacer les séquences GAG/POL dans la construction décrite au paragraphe C, où les séquences Poly-A de SV40 sont maintenues. En pratique, le LTR viral est excisé de la construction décrite au paragraphe a) par une digestion BglII qui libère une extrémité cohésive pour le récepteur à l'acide rétinoique (BamHI en 3', cohésif de BglII).

La construction RARβ enveloppe amphotrope peut aussi être réalisée comme suit : la construction mise en oeuvre a utilisé un raccourci expérimental mettant à profit la construction LTR-env ampho-SV40 polyA. Le clonage a été réalisé comme suit : le fragment BglII (position - 747)-BamHI (position + 155) de 902 bases du promoteur β de l'acide rétinoïque incluant l'ensemble des éléments régulateurs de la transcription fonctionnellement utiles a été purifié et cloné dans un plasmide pUC19 dans lequel un linker BglII a été placé en HindIII. Les fragments XmnI (coupure à l'intérieur du plasmide pUC19)-BglII dérivés de la construction sus-mentionnée et de la construction LTR-env-AMPHO (on retient le fragment contenant les séquences d'enveloppe) sont associés par ligation. L'orientation se fait sur le site EcoRI du fragment RAR-β ; l'orientation est possible grâce à la présence d'un autre site EcoRI dans l'enveloppe Ampho.

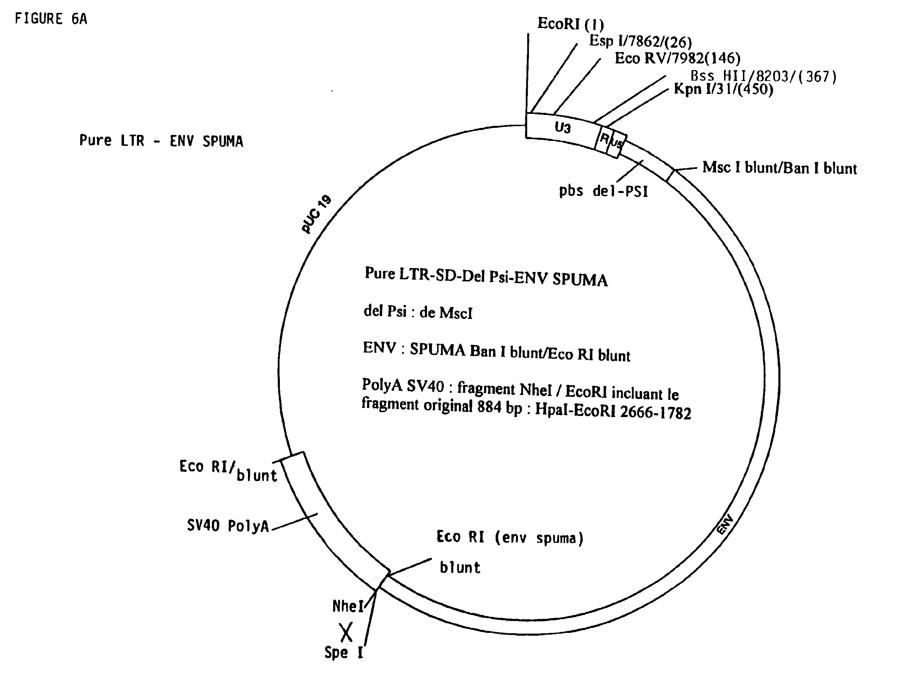

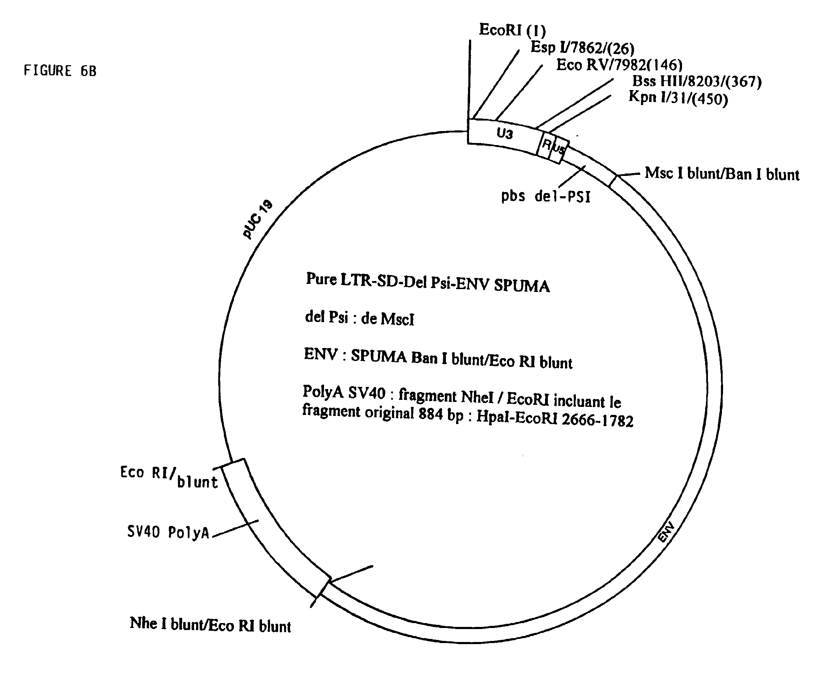

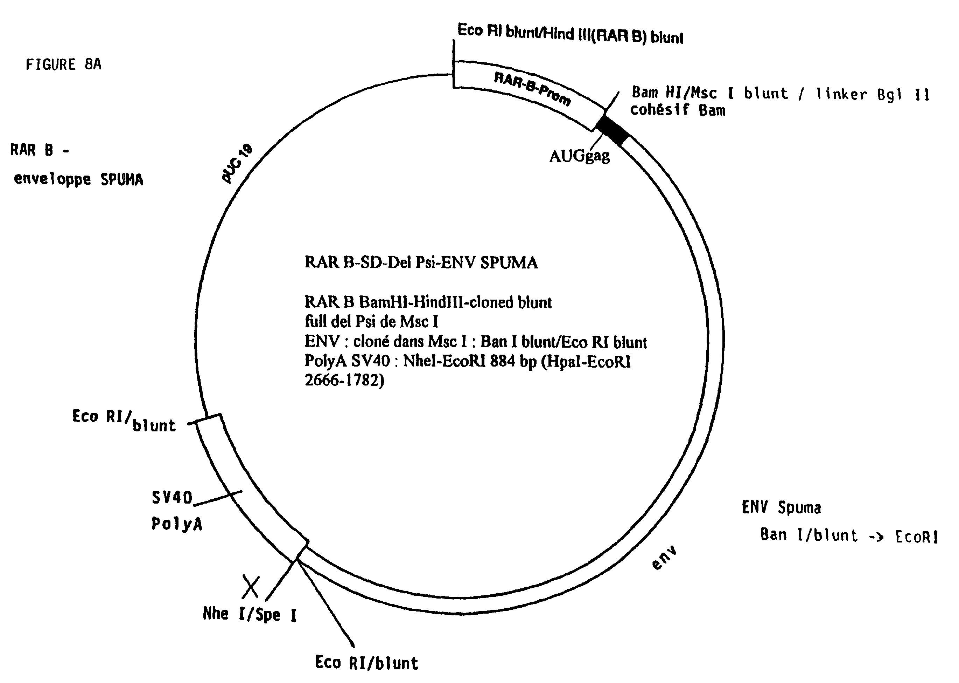

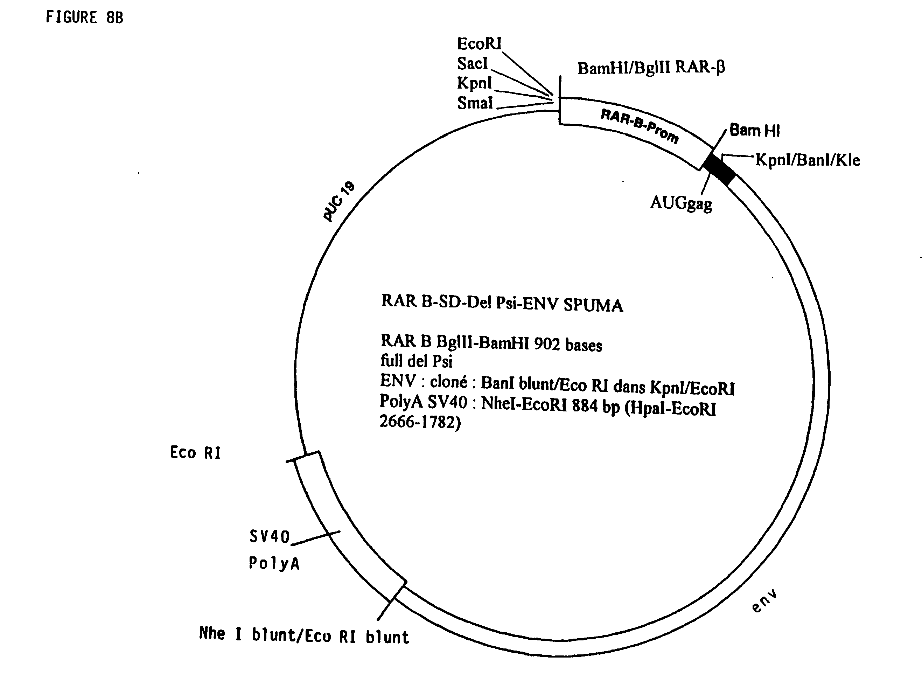

### 3) Construction ENV de spumavirus (Figures 6 et 8)

Dans un deuxième exemple, les séquences d'enveloppe de spumavirus ont été utilisées.

Les séquences d'enveloppe de spumavirus viennent remplacer les séquences d'enveloppe amphotrope 4070A des constructions sus-mentionnées : où les séquences Poly-A de SV40 sont maintenues.

Un fragment génomique EcoRI-EcoRI de spumavirus est sous cloné en EcoRI de pUC19. Le fragment utilisé dans les constructions exprimant l'enveloppe est obtenu par digestion BanI, ménageant 30 bases en amont de l'ATG de l'enveloppe dans le génome de spumavirus ; en conservant le site EcoRI en aval, proche du signal de terminaison (Flugel RM et al., 1987).

Comme pour l'enveloppe amphotrope, deux options sont retenues :
a) Transcription sous 5'LTR viral avec site de polyadénylation d'aval de SV40. Pour la construction LTR-enveloppe spumavirus, le fragment EcoRI-EcoRI, traité à la Klenow, de l'enveloppe spumavirus est cloné en HindIII/Klenow-SmaI à la place de l'extrémité de POL dans le pUC18 fin POL/polyA-SV40. Le fragment BanI-EcoRI contenant les séquences de l'enveloppe spuma suivie du poly de SV40 est ensuite excisé et cloné (blunt : traitement à la Klenow) aux sites Mscl/HindIII (excision des séquences d'necapsulation et GAG) du plasmide 5'LTR-FB29 pure-LTR. L'orientation se fait par une digestion PvuII.
b) Transcription sous promoteur inductible par un traitement pharmacologique, en particulier le promoteur sus-décrit du récepteur beta à l'acide rétinoique : Pour la construction RAR-β promoteur-enveloppe spumavirus, le fragment BanI-EcoRI sus-mentionné contenant les séquences de l'enveloppe spuma suivie du poly de SV40 est cloné dans un plasmide pUC18 aux sites KpnI-EcoRI. Le site BamHI du polylinker est ensuite ouvert et le promoteur RAR-β y est cloné (fragment BglII-BamHI dont les deux extrémités sont cohésives BamHI). L'orientation est à nouveau établie à partir du site EcoRI dégageant deux fragments très asymétriques dans le promoteur RAR-β.

A chacune des étapes, pour chacune des constructions envisagées, un clone cellulaire est retenu selon l'intensité de synthèse des proteines virales de complémentation et la stabilité ; une "Master Cell Bank" est préparée pour chaque lignée sélectionnée.

### D) CONSTRUCTION DES LIGNEES D'ENCAPSIDATION A PARTIR DE LA CELLULE FOETALE DE CHIEN "DOG"

### D.1. Sélection des cellules foetales de chien

La sélection des cellules foetales de chien est réalisée par mise en oeuvre des étapes suivantes :
a) culture de cellules eucaryotes choisies sur milieu riche ISCOVE contenant 10% de sérum de veau foetal et 10% de sérum de cheval,
b) passage des cellules cultivées en milieu DMEM contenant 20% de sérum de veau foetal et culture des cellules pendant un mois dans un milieu sans CO₂, récupération des cellules sélectionnées,
c) sélection des cellules présentant une cinétique accélérée,
d) passage des cellules sélectionnées en milieu DMEM contenant 10% de sérum de veau foetal,
e) passage des cellules récupérées à l'étape d) en milieu DMEM contenant 10% de sérum de veau nouveau né,
f) récupération des cellules sélectionnées à l'issue de l'étape e).

### D.2. Préparation des lignées d'encapsidation

### a) Transfection de la lignée DOG par les séquences gag/pol (obtention de DOGP29).

La méthode de transfection utilise la précipitation au phosphate de calcium selon la procédure standard, sans ADN carrier : 10 microgrammes de plasmide ont été déposés sur une boîte de culture de 35 mm de diamètre où 5.10⁴ cellules ont été plantées la veille.

Les cellules sont cultivées en milieu de Dulbecco modifié Eagle (DMEM, Gibco-BRL) additionné de 10% de sérum de veau nouveau-né (HyClone). Deux jours après la transfection, les cellules sont trypsinées, diluées au 1/20ème et mises sous sélection. Les colonies apparues après 12 jours sont prélevées et réimplantées sur des boîtes de culture de 24 puits, à raison d'un clone par puits.

DOGP29 a été obtenue par transfection de LTR-SD-Délétion Psi-GAG / POL dont on a décrit la construction détaillée ci-dessus (et co-transfection avec gène de sélection, résistance à la phléomycine) sur cellule foetale de chien optimisée selon les critères suivants : 1- Absence de rétrovirus endogènes ; 2-Cellule adhérente ; 3- Croissance rapide ; 4-Morphologie stable et homogène ; 5-aisément transfectable ; 6- Nombre de passages supporté très élevé (passages artificiels intensifs pour test) ; 7-Eventuellement capable de soutenir LTC-IC (Hémato).

Un master Cell Bank System est réalisé à partir du clone de cellules de chien retenu selon l'intensité de synthèse des protéines virales de complémentation et la stabilité d'expression de la reverse transcriptase (POL) .

L'algorithme d'évaluation suivant a été utilisé pour évaluer les lignées d'encapsidation

### Evaluation primaire : - preuve d'efficacité

**Evaluation secondaire** : - sélection de lignées stables avec le taux d'expression le plus approprié des protéines virales transcomplémentant le vecteur rétroviral défectif transportant le gène d'intérêt.

Dans les deux situations la méthodologie est la même mais dans le second cas interviennent les notions d'évaluation quantitative et de stabilité.

### Stratégie d'évaluation, combinatoire :

### 1°/ Mise en évidence moléculaire de la présence des séquences d'intérêt

Ceci repose sur la technologie de la réaction en chaîne de la polymérase (PCR) utilisant des amorces spécifiques des séquences codant pour les protéines virales trans-complémentantes. Les amorces utilisées sont les suivantes :

### Séquences GAG (souche FB29 du virus de Friend)

| | | | | | | | | |
|---|---|---|---|---|---|---|---|---|
| JB 2008-S | TGA | CGG | GAG | AAG | AAA | AAC | AGC | G |
| GAG-R | TAG | GAG | CAA | CTG | GCG | ATA | GTG | G |

### Séquences POL (souche FB29 du virus de Friend)

| | | | | | | | | |
|---|---|---|---|---|---|---|---|---|
| POL-F | GAT | TGC | CAG | ACT | TGA | CTA | AGC | C |
| POL-R | TAC | CAT | CCG | TAG | GCA | AGG | | |

### Séquences ENV (amphotrope (4070A du virus de Moloney)

| | | | | | | | | |
|---|---|---|---|---|---|---|---|---|
| AMPHO-F | ATA | CCA | ATC | ATT | CCA | CCG | CTC | C |
| AMPHO-R | GTT | GAG | GTC | TGT | CTG | GAT | AGC | G |

### Séquences ENV (spumavirus HFV ou HSRV)

| | | | | | | | | |
|---|---|---|---|---|---|---|---|---|
| HFVENV-F | TAG | GAC | CTG | TAA | TAG | ACT | GG | |
| HFVENV-R | CCT | TGA | GAA | CTC | CAA | TCC | TTC | G |

### 2°/ Vérification fonctionnelle de la construction de base destinée à exprimer les protéines GAG/POL et délétée du signal d'encapsidation

- Transfection des constructions GAG/POL dans les cellules DOGP29 ; en co-transfection avec un plasmide porteur d'un gène de sélection.
- Expression de POL : elle est vérifiée par un test d'activité transcriptase inverse (reverse transcriptase)
- Expression de GAG : elle est vérifiée par western blot sur lysats cellulaires : l'anticorps utilisé est un sérum polyclonal de chèvre spécifique de la protéine GAG-CAp30 (selon les techniques habituelles)

### 3°/ Inductibilité de l'expression des séquences mises sous dépendance du promoteur β de l'acide rétinoïque

- Les cellules sont traitées avec de l'acide tout-trans rétinoïque, à la concentration de 10⁻⁶ M ; l'effet inducteur est au mieux évalué dans les 24 à 48 heures qui suivent le traitement.

### 4°/ Pseudotypage de particules virales, vecteurs rétroviraux défectifs dérivés de virus murins (en particulier virus leucémogènes de Moloney ou de Friend), par l'enveloppe de spumavirus

### EXPRESSION de l'enveloppe de spumavirus par les lignées de packaging :

- Western blot utilisant un anticorps spécifique de l'enveloppe de HSRV
- Immunofluorescence sur les cellules exprimant l'enveloppe

### INFECTION de cellules cibles :

- transmission de séquences génétiques transportées par un vecteur rétroviral défectif dérivé de virus murins (en particulier virus leucémogènes de Moloney ou de Friend), à des cellules naives par infection virale médiée par l'enveloppe de HSRV.

Les meilleurs clones DOG gag/pol (DOGP29) sont sélectionnés par dosage de l'activité Réverse Transcriptase selon le protocole suivant dont seules les particularités sont données, les autres conditions étant connues de l'homme du métier :
Les échantillons comprenant le surnageant des cellules DOGP29 à tester sont déposés dans une plaque 96 puits à fond rond sous 50 *µ*l (pipette multicanaux).

On utilise les témoins de radioactivité : H₂O, VIH +, témoins cellules ou surnageant culture lymphocytes de donneur.

On ajoute alors 10 *µ*l de tampon A(KCl=0, 5M DTT = 50 mM Triton x 100 = 0,5 %).

Puis 40 *µ*l de tampon B (10*µ*l EGTA 5mM dans TRIS HCl 0.5 M pH 7.8 1*µ*l MgCl₂ 0,5 M, 3 *µ*l ³HdTTP (1 mCi /ml) 10 µl poly rA - oligo dT 5 OD/ml 16µl d'eau).

On incube 1 heure à l'étuve à 37°C. On rajoute 20µl de tampon C [Na₄P₂O₇ 120 mM dans TCA 60%]. On incube 15 minutes à +4 °C puis on allume le poupinel à 60°C. La pompe à vide, l'injecteur et le SKATRON (filtre) sont mis en marche. On vérifie les volumes. On place l'alcool sur le canal 2, l'eau sur le canal 1, le décontaminant sur le canal 3. On met ensuite le skatron en position "MANUEL" et rincer :
1. canal 3 : décontaminant (TFD 4 à 2%) pendant 2 fois 5 secondes.
2. air
3. alcool : 20"
4. air
5. eau distillée : 20 à 30 secondes.

Le bouchon de l'eau est mis sur le TCA, et celui d'alcool sur l'eau.

Les filtres sont séchés dans le poupinel à 60°C.

On découpe alors les filtres et on les dépose dans des tubes préparés à cet effet.

2 ml de liquide de scintillation sont ensuite distribués.

Les tubes sont conservés à +4°C si on ne peut les lire immédiatement.

Les analyses sont interprétées par ordinateur :

les résultats sont fournis en coup par minute. Le compteur peut aussi fournir la moyenne de quatre puits.

| Tampon A | |
|---|---|
| KCl (Chlorure de potassium) 0,5 M | 7,45g |
| DTT (dithiothreitol) 50 mM | 1,54 g |
| Triton 100X ->0,5% | lml |
| Eau distillée stérile qsp | 200 ml |
| Conservation +4°C Pou 1 test : | 10µl |

| Tampon B | |
|---|---|
| EGTA 5 mM dans TRIS 0,5 M pH7,8 | 10 *µ*l |
| MgCl2 0,5 M | 1*µ*l |
| 3HdTTP (1 mCi/ml) | 3*µ*l |
| poly rA -oligo dT 5 OD/ml | 10 *µ*l |
| eau distillée stérile | 16*µ*l |

Préparer extemporanément la quantité nécessaire, soit pour 1 test, 40 *µ*l.

Tampon C
(Na4P2O7 120 mM dans TCA 60%)

Peser 10,7 g de PpNa (=pyrophosphate de sodium = Na4P2O7, 10 H2O)
Ajouter 120 g de TCA (Acide trichloracétique)
qsp 200 ml d'eau distillée stérile
Conservation +4°C.

Tampon de lavage SKATRON
(Pyrophosphate de Sodium 1,2 mM dans TCA 5%).

Peser 21,4 g de Na4P207 (10 H2O) (Pyrophosphate de Sodium)
Ajouter 200 mg de TCA pur
qsp 4 litres (soit 3800 ml) d'eau distillée stérile.

### Protocole optimisé de test de l'activité réverse transcriptase

Une procédure optimisée pour la détection de l'activité transcriptase inverse des virus leucémogènes murins consiste à réaliser la réaction de polymérisation non pas dans un tampon contenant du chlorure de Magnésium (tel que décrit ci-dessus et conformément aux conditions indiquées dans les catalogues des fournisseurs de produits de Biologie Moléculaire) mais en présence de Manganèse (Maniatis, Molecular Biology, a Laboratory Manual, Cold Spring Harbor, 1990).

Le protocole est le suivant : mélanger 50 µl de surnageant de culture à tester pour l'activité transcriptase inverse, avec 50 *µ*l d'une solution 2x comportant les éléments suivants : Tris pH 8.3 0.1 M ; DTT 25 mH ; MnC12 1.2 mM ; Nacl 125 mM ; NP40 1/1000 ; dTTP 0,02 mM ; dTTP alpha-32 P (SA : 3000 Ci/mmole) 10 *µ*Ci/ml ; Oligo dT-Poly rA 20 *µ*g/ml ; H₂O qsp. Une incubation de 3 heures à 37°C est pratiquée. Chaque réaction est filtrée sur un papier Whatman DE81, à l'aide d'un appareil à slot-blot ; deux épaisseurs de papier Whatman 3MM étant placées sous le DE81. Plusieurs lavages sont réalisés 3 x en 2x SSC ; sous slot-blot ; puis, lavage du filtre lui-même 3 x 10 minutes en 2x SSC. Enfin le filtre est rincé 2 x 1 min en EtOH 95% et séché aussitôt. Une exposition est pratiquée en présence d'écran amplificateur.

### b) Transfection de la lignée DOGP29 par la séquence env.

La transfection est réalisée dans les conditions décrites pour la construction gag-pol.
Deux constructions différentes ont été réalisées :
- l'une (DOGP29L pour LTR) où l'expression de l'enveloppe de spumavirus est pilotée par le LTR de FB29
- l'autre où l'expression est pilotée par un promoteur inductible, en particulier, promoteur au récepteur-β de l'acide rétinoïque (éventuellement associé à l'enhancer du gène Thymidine- kinase d'HSV, si l'expression d'enveloppe s'avérait insuffisante).

A titre d'exemple, le promoteur du récepteur β à l'acide rétinoïque est utilisé (promoteur RAR-β).

C'est un fragment HindIII en 5' et BamHI en 3' du récepteur β à l'acide rétinoïque qui est utilisé dans l'ensemble des contructions réalisées (de Thé et al., 1990). A partir de la construction décrite ci-dessus, un linker BglII est introduit au site MscI de FB29, et le fragment HindIII en 5'-BamHI en 3' du promoteur du récepteur β à l'acide rétinoïque est placé en amont des séquences GAG-POL dépourvues du signal d'encapsidation.

### c) Sélection des clones DOG29 qaq/pol/env pour l'enveloppe de spumavirus.

Cette sélection est réalisée par un test d'immunofluorescence mettant en oeuvre des anticorps anti-env spumavirus.

Dans une première étape, on réalise des coupes histologiques puis on les soumet à une analyse FACS (Fluorescence-activated cell sorter).

Une sélection peut également être réalisée par la technique de Western blot.

### d) Tests fonctionnels d'infectivité pour les lignées gag/pol et env.

Les tests d'infectivité supposent une étape de transfection avec un vecteur rétroviral défectif incluant un gène de sélection. Un tel vecteur a été décrit dans la partie A.

### - Infection des fibroblastes NIH3T3

L'infection a été réalisée avec le surnageant de culture de cellules d'un puits parvenu à confluence conformément à la technique décrite à la partie A-4.

### - Réaction en chaîne de la polymérase (PCR)

Elle a été effectuée selon la technique décrite au point 5 de la partie A.

Les oligonucléotides utilisés sont les suivants:
5'-TAAGCAATTCGGTGGGGTCTTTCATTG-3'(oligonucléotide sens) et 5'-CTGCTGACGGGAGAAGAAAAAC-3'(oligonucléotide antisens).

### - Détermination des titres infectieux après transfection d'un vecteur rétroviral défectif incluant un gène de sélection.

La méthode employée a été exposée au point 6 de la partie A ci-dessus à l'exception de l'étape de sélection qui s'est déroulée comme suit : 20 heures après l'infection, les cellules sont mises sous sélection par addition de Généticine (1 mg/ml) au surnageant.

### - Nombre de copies passées

En complément à la titration standard sur NIH3T3 a été proposée une autre technique de titration, permettant notamment de répondre à la situation où les vecteurs rétroviraux optimisés ne véhiculent pas de gène de sélection à l'intérieur de la construction. La titration repose alors sur des méthodes moléculaires, en particulier, quantification du nombre de copies du vecteur rétroviral passées en moyenne en utilisant un southern blot. Ce southern porte l'ADN de cellules infectées par le vecteur hydrolyse par une enzyme de restriction choisie pour couper dans chacun des LTRs du vecteur rétroviral complémenté par la lignée (ex:KpnI); des dilutions adéquates de plasmides portant le rétrovirus considéré digéré par la même enzyme de restriction, sont juxtaposées sur le même blot.

### SOUTHERN BLOT

Deux jours après l'infection par du surnageant pur, les NIH3T3 sont trypsinées passées au 1/20^{ème} environ sur trois boîtes de culture de 100 mm de diamètre, dont l'une est mise sous sélection par la généticine. A confluence, l'ADN génomique des cellules infectées par chacun des deux clones après ou sans sélection, est extrait puis quantifié. L'ADN a été digéré par deux enzymes de restriction, PstI et KpnI afin de réaliser un Southern blot. Après contrôle de la qualité de la digestion et homogénéïsation de la quantité d'ADN déposée dans chaque puits, le transfert a été réalisé sur une membrane de Nylon Hybond N (Amersham). L'hybridation a été conduite avec une sonde incluant la totalité des séquences du LTR viral encadrées par 100 bases en amont et 100 bases en aval. La sonde a été marquée par extension d'amorce (Feinberg et Vogelstein, 1983, 1984) avec du dCTP marqué à l'alpha-32-P, à une activité spécifique de 5*10⁸ cpm/ug. L'hybridation a été pratiquée dans un milieu constitué de : 50% formamide désionisée ; 5X SSEP ; 1X denhardt's ; 5% dextran sulfate et 0,2mg/ml d'ADN de saumon soniqué, pendant 20 heures à 42°C. De brefs rinçages ont été pratiqués dans une solution peu stringente 2X SSEP/0,1% SDS, 5min à température ambiante et 10 min à 65°C ; suivis d'une exposition de 3 jours sur films Kodak-XAR-5 à -80°C avec écrans amplificateurs Li-Plus (Dupont-NEN).

### - Recherche de production de virus Helper

La technique de recherche est décrite au point 7 de la partie A ci-dessus.

### REFERENCES

ANDERSON WF, MCGARRITY GJ, MOEN RC. Report to the NIH Recombinant DNA Advisory Committeee on murine Replication- Competent Retrovirus (RCR) assays (February 17, 1993). *Hum Gene Ther,* **4 :** 311-321, 1993
CHATTOPADHYAY et al. *J Virol.,* **39 :** 777, 1981
COHEN-HAGUENAUER O. Regulation of gene therapy in Europe : a current statement with référence to US regulation *Eur J of Cancer,* 1994 ; **30A :** 1193-1201 CORNETTA K, MORGAN RA, ANDERSON WF. Safety issues related to retroviral-mediated gene transfer in humans. *Hum. Gene Ther.* **2** : 5-14, 1991
DANOS O, MULLIGAN RC. Safe and efficient generation of recombinant retroviruses with amphotropic and ecotropic host ranges *Proc Natl Acad Sci USA* **85 :** 6460-64, 1988
DANOS O. Construction of retroviral packaging cell lines In *"Methods in Molecular Biology, : Practical Molecular Virology* : viral vectors for gene expression" Collins M Ed., the Humana Press Inc., Clifton, NJ, 1991, **8**, pp 17-27
DONAHUE RE, KESSLER SW, BODINE DET AL. Helper virus induced T cell lymphoma in non-human primates after retroviral-mediated gene transfer. J *Exp Med,* **176**, 1125-1135, 1992
FLUGEL RM, RETHWILM A, MAURER B, DARAI G. Nucleotide sequence analysis of the env gene and its flanking regions of the human spumaretrovirus reveals two novel genes. *EMBO* **6** : 2077-2084, 1987
FRENCH ANDERSON W Human gene Therapy Science **256 :** 808-813, 1992
GUNTER KC, KHAN AS, NOGUSHI PD. The safety of retroviral vectors. *Hum Gen Ther,* **4**, 643-645, 1993
KESSLER DA, SIEGEL JP, NOGUSHI PD, ZOON KC, FEIDEN KL, WOODCOCK J. Regulation of Somatic-cell therapy and gene therapy by the food and drug administration *N Engl J Med,* **329** : 1169-1173, 1993
MATHIEU-MAHUL D, HEARD JM, FICHELSON S, GISSELBRECHT S, SOLA B, LARSENC Viral expression in two myelomonocytic cell lines obtained from long-term bone marrow culture infected with the Friend polycythemia-inducing virus (FV-P) *Virology* **119** : 59-67, 1982
McLACHLIN JR, CORNETTA K, EGLITIS MA, ANDERSON WF.Retroviral- mediated gene transfer *Progress in Nucleic Acid Research and Molecular Biology* **38** : 91-135, 1990
MILLER AD.Human gene therapy comes of age *Nature* **357 :** 455- 460, 1992
MULLIGAN RC. The basic science of gene therapy *Science* **260** : 926-931, 1993
PERRYMAN S, NISHIO J, CHESEBRO B Complete nucleotide sequence of Friend murine leukemia virus, strain FB29 *Nucl Acids Res* **19** : 6950, 1991
RESTREPO LM, DAL CORTIVO L, HEARD JM, MAROLLEAU JP, MARTY M, BOIRON M, COHEN-HAGUENAUER O, Transduction of haemopoietic progenitors with an original retroviral vector derived from Fr- MuLV with high infection efficiency (soumis, Blood)
SITBON M, SOLA B, EVANS L, NISHIO J, HAYES SF, NATHANSON K, GARON CF, CHESEBRO B. Hemolytic anemia and erythroleukemia, two distinct pathogeneic effects of Friend MuLV : mapping of the effects to different regions of the viral genome *Cell* **47** : 851- 859, 1986
SITBON M, ELLERBROK H, POZO F, NISHIO J, HAYES SF, EVANS LH, CHESEBRO B. Sequences in the U5-gag-pol region influence early and late pathogenic effects of Friend and Moloney murine leukemia viruses *J Virol* **64:** 2135-40, 1990
SITBON M, D'AURIOL L, ELLERBROK H, ANDRE C, NISHIO J, PERRYMAN S, POZO F, HAYES SF, WEHRLY K, TAMBOURIN P, GALIBERT F, CHESEBRO B. Substitution of leucine for isoleucine in a sequence highly conserved among retroviral envelope surface glycoproteins attenuates the lytic effect of Friend murine leukemia virus *Proc Natl Acad Sci USA* **88** : 5932-5936, 1991
DE THE H, VIVANCO-RUIZ MM, TIOLLAIS P, STUNNENBERG H, DEJEAN A. Identification of a retinoic acid responsive element in the retinoic acid receptor β gene. Nature 49 : 177-180, 1990
TEMIM HM. Safety considerations in somatic gene therapy of human disease with retrovirus vectors. *Hum Gene Ther,* **1 :** 11-123, 1990
BUJARD H., Gene Therapy 2 : S1, 1995
   BURNS JC et al., Proc. Natl. Acad. Sci. USA 90 : 8033-8037, 1993
   CHATTOPADHYAY SK. et al., J. Virol. 39 : 777-791, 1981 EMI N. et al., J. Virol 65 : 1202-1207, 1991
   MILLER D. et al., J. Virol. 65 :2220-2224, 1991
   WANG Y. et al., Proc. Natl. Acd. Sci. USA 91 : 8180-8184, 1994
   WILSON C. et al., J. Virol. 63 : 2374-2378, 1989
   ZAWADA J. et al., J. Gen. Virol. 15 : 183-191, 1972.

## Revendications

1. Cellule eucaryote pour l'encapsidation par transcomplémentation, d'ARN rétroviraux recombinants permettant le transfert et/ou l'intégration au sein du génome d'une cellule cible, d'une séquence de nucléotides choisie (séquence transgénique), ladite cellule étant sélectionnée parmi les cellules foetales ou embryonnaires de chien, telle qu'obtenue par sélection conformément à la méthode suivante :
a) culture de cellules eucaryotes choisies sur milieu riche ISCOVE (GIBCO) contenant en outre 10% de sérum de veau foetal et 10% de sérum de cheval,
b) passage des cellules cultivées en milieu DMEM contenant 20% de sérum de veau foetal et récupération des cellules sélectionnées et culture des cellules pendant un mois dans un milieu sans CO₂,
c) sélection des cellules ayant un taux de multiplication rapide (cinétique accélérée),
d) passage des cellules sélectionnées en milieu DMEM contenant 10% de sérum de veau foetal,
e) passage des cellules récupérées à l'étape d) en milieu DMEM contenant 10% de sérum de veau nouveau né (HyClone),
f) récupération des cellules sélectionnées à l'issue de l'étape e) ; et ladite cellule étant **caractérisée en ce qu'**elle vérifie les propriétés suivantes
- elle a une morphologie homogène et stable dans le temps,
- elle n'est pas d'origine tumorale,
- elle a la capacité d'être sélectionnée dans un milieu de culture minimum et dépourvu de CO₂, sans transformation préalable
- elle a un taux de multiplication rapide.

2. Cellule selon la revendication 1, **caractérisée en ce qu'**il s'agit d'une cellule foetale de chien DOGOM1 déposée à la CNCM le 30 novembre 1994 sous le numéro I-1496.

3. Cellule recombinante permettant l'encapsidation d'ARN rétroviraux recombinants par transcomplémentation (cellule recombinante transcomplémentante), **caractérisée en ce qu'**il s'agit d'une cellule selon l'une quelconque des revendications 1 ou 2, modifiée par exemple par transfection ou par infection avec les séquences de nucléotides codant pour les polypeptides ou glycoprotéines de transcomplémentation, lesdites séquences étant apportées au moyens de deux vecteurs au moins.

4. Cellule recombinante permettant l'encapsidation d'ARN rétroviraux recombinants par transcomplémentation (cellule recombinante transcomplémentante), **caractérisée en ce qu'**il s'agit d'une cellule selon l'une quelconque des revendications 1 à 3, génétiquement modifiée par exemple par transfection ou infection avec :
1) un premier vecteur d'expression pour la complémentation en enveloppe d'un vecteur rétroviral permettant le transfert et/ou l'intégration au sein du génome d'une cellule cible, d'une séquence de nucléotides choisie (dite "séquence transgénique"), ledit vecteur pour la complémentation comprenant une séquence de nucléotides codant pour les polypeptides d'enveloppe et des éléments de régulation de l'expression de cette séquence,
2) un deuxième vecteur d'expression pour la transcomplémentation d'un vecteur rétroviral permettant le transfert et/ou l'intégration au sein du génome d'une cellule cible, d'une séquence de nucléotides choisie (dite "séquence transgénique"), comprenant :
a) une séquence de nucléotides dite séquence gag codant pour les polypeptides dérivés d'une nucléoprotéine (polypeptides gag) d'un rétrovirus du type du rétrovirus de Friend,
b) une séquence de nucléotides dite séquence *pol* codant pour les polypeptides dérivés dont une protéine transcriptase inverse et une intégrase (polypeptides pol) d'un rétrovirus du type du rétrovirus de Friend,
c) des signaux régulateurs de transcription contrôlant l'expression des séquences *gag* et *pol,*
les susdits premier et deuxième vecteurs d'expression étant dépourvus de signaux d'encapsidation,

5. Cellule recombinante transcomplémentante **caractérisée en ce qu'**il s'agit d'une cellule selon l'une quelconque des revendications 1 à 3, et **en ce qu'**elle comprend :
a) une séquence de nucléotides dite séquence gag codant pour les polypeptides dérivés d'une nucléoprotéine (polypeptides gag) d'un rétrovirus du type du rétrovirus de Friend,
b) une séquence de nucléotides dite séquence *pol* codant pour les polypeptides dérivés d'une transcriptase inverse et d'une intégrase (polypeptides pol) d'un rétrovirus du type du rétrovirus de Friend,
c) des signaux régulateurs de transcription contrôlant l'expression des séquences gag et *pol,* et
d) une séquence de nucléotides dite séquence *env*, codant pour les polypeptides dérivés de l'enveloppe (polypeptides env) d'un rétrovirus de la famille des spumavirus, les polypeptides env permettant l'encapsidation d'ARN rétroviraux,
e) des signaux régulateurs de transcription contrôlant l'expression de la séquence *env*.

6. Cellule recombinante transcomplémentante selon la revendication 5, **caractérisée en ce qu'**il s'agit de la cellule DOGOM1 déposée à la CNCM sous le numéro I-1496, recombinée d'une part avec les séquences nucléotidiques *gag et pol* de la souche FB29 du rétrovirus de Friend, sous le contrôle des signaux de transcription contenus dans la séquence LTR de la souche FB29 du rétrovirus de Friend et recombinée d'autre part avec la séquence de nucléotides *env* d'un rétrovirus du type spumavirus sous le contrôle d'un promoteur inductible, par exemple le promoteur RAR-β.

7. Cellule recombinante transcomplémentante selon la revendication 6, **caractérisée en ce qu'**il s'agit de la cellule DOGOM1 déposée à la CNCM sous le numéro I-1496, recombinée d'une part avec les séquences nucléotidiques *gag* et *pol* de la souche FB29 du rétrovirus de Friend, sous le contrôle d'un promoteur non viral, par exemple d'un promoteur inductible tel que le promoteur RAR-β, ou d'un promoteur conditionnel.

8. Cellule recombinante transcomplémentante **caractérisée en ce qu'**il s'agit d'une cellule selon l'une quelconque des revendications 1 à 3, et **en ce qu'**elle comprend :
a) une séquence de nucléotides dite séquence gag codant pour les polypeptides dérivés d'une nucléoprotéine (polypeptides gag) d'un rétrovirus du type du rétrovirus de Friend,
b) une séquence de nucléotides dite séquence *pol* codant pour les polypeptides dérivés d'une transcriptase inverse et d'une intégrase (polypeptides pol) d'un rétrovirus du type du rétrovirus de Friend,
c) des signaux régulateurs de transcription contrôlant l'expression des séquences gag et *pol,* par exemple des signaux non viraux, et
d) une séquence de nucléotides dite séquence *env*, codant pour les polypeptides dérivés d'une enveloppe amphotrope, par exemple l'enveloppe 4070A du virus de la leucémie de Moloney (Mo-MuLV),
e) des signaux régulateurs de transcription non viraux, contrôlant l'expression de la séquence *env,* contenant par exemple un promoteur inductible ou conditionnel, par exemple le promoteur RAR-β.

9. Cellule recombinante transcomplémentante selon l'une quelconque des revendications 4 à 8, **caractérisée en ce que** la séquence de nucléotides du vecteur d'expression pour la transcomplémentation, codant pour les polypeptides d'enveloppe est modifiée, par exemple par remplacement de nucléotides par un enchaînement de nucléotides codant pour une séquence d'un polypeptide ou d'une glycoprotéine reconnue spécifiquement par un type cellulaire défini, ou par l'addition d'un tel enchaînement.

10. Cellule recombinante transcomplémentante selon l'une quelconque des revendications 4 à 9, **caractérisée en ce qu'**elle est en outre transfectée par un vecteur rétroviral pour l'expression et/ou le transfert et/ou l'intégration au sein du génome d'une cellule cible, d'une séquence de nucléotides choisie ("séquence transgénique"), ledit vecteur rétroviral étant le vecteur pFOCH29 représenté à la figure 1 et déposé à la CNCM sous le n°I-1326 dans lequel est inséré en un site non essentiel pour sa réplication, la susdite séquence transgénique.

11. Cellule recombinante transcomplémentante selon l'une quelconque des revendications 5 à 9, **caractérisée en ce que** le vecteur rétroviral est dérivé du vecteur pFOCH29 déposé à la CNCM sous le n° I-1326, notamment par délétion au niveau de la séquence LTR (vecteur pFOCH29PL représenté à la figure 2).

12. Cellule recombinante transcomplémentante selon l'une quelconque des revendications 4 à 11, **caractérisée en ce que** la séquence transgénique code pour un gène à visée thérapeutique, ou pour une séquence de nucléotides antisens, ou pour un mutant négatif dominant d'un gène donné ou une séquence codant pour un inhibiteur fonctionnel d'un gène donné pour un gène marqueur ou pour une séquence de régulation d'un gène donné ou pour un gène susceptible d'ajouter une fonction nouvelle à la cellule cible.

## Claims

1. Eukaryotic cell for encapsidation by transcomplementation of recombinant retroviral RNAs enabling the transfer and/or integration within a target cell genome of a chosen nucleotide sequence (transgenic sequence), said cell being selected from among foetal or embryonic cells of the dog, as obtained by selection in accordance with the following method:
a) culture of chosen eukaryotic cells on ISCOVE rich medium (GIBCO) also containing 10% foetal calf serum and 10% horse serum,
b) passage of the cultured cells in DMEM medium containing 20% foetal calf serum and recovery of the selected cells and culture of the cells for one month in medium without CO₂,
c) selection of the cells having a rapid multiplication rate (accelerated kinetics),
d) passage of the selected cells in DMEM medium containing 10% foetal calf serum,
e) passage of the cells recovered in step d) in DMEM medium containing 10% neonatal calf serum (HyClone),
f) recovery of the cells selected at the end of step e); and said cell being **characterised in that** it is confirmed in the following properties:
- it has a morphology that is homogeneous and stable over time,
- it does not have a tumour origin,
- it has the capacity to be selected in minimal culture medium, without CO₂, and without prior transformation
- it has a rapid multiplication rate.

2. Cell as claimed in claim 1, **characterised in that** it is a foetal dog cell DOGOM1 registered with the CNCM on 30 November 1994 under the number I-1996.

3. Recombinant cell enabling encapsidation of recombinant retroviral RNAs by transcomplementation (transcomplementing recombinant cell), **characterised in that** it is a cell as claimed in any of claims 1 or 2, modified for example by transfection or by infection with nucleotide sequences coding for transcomplementation polypeptides or glycoproteins, said sequences being supplied by means of at least two vectors.

4. Recombinant cell enabling encapsidation of recombinant retroviral RNAs by transcomplementation (transcomplementing recombinant cell), **characterised in that** it is a cell as claimed in any of claims 1 to 3, genetically modified for example by transfection or infection with:
1) a first expression vector for complementation in the envelope of a retroviral vector enabling the transfer and/or integration within a target cell genome of a chosen nucleotide sequence (referred to as "transgenic sequence"), said vector for complementation including a nucleotide sequence coding for the envelope polypeptides and elements for regulation of the expression of this sequence,
2) a second expression vector for the transcomplementation of a retroviral vector enabling the transfer and/or integration within a target cell genome of a chosen nucleotide sequence (referred to as "transgenic sequence"), including:
a) a nucleotide sequence designated the gag sequence coding for polypeptides derived from a nucleoprotein (gag polypeptides) of a retrovirus of the Friend retrovirus type,
b) a nucleotide sequence designated the *pol* sequence coding for polypeptides derived from a reverse transcriptase protein and an integrase (pol polypeptides) of a retrovirus of the Friend retrovirus type.
c) transcription regulatory signals controlling the expression of the *gag* and *pol* sequences,
the aforesaid first and second expression vectors being free of encapsidation signals.

5. Transcomplementing recombinant cell **characterised in that** it is a cell as claimed in any of claims 1 to 3, and **in that** it comprises:
a) a nucleotide sequence designated the gag sequence coding for polypeptides derived from a nucleoprotein (gag polypeptides) of a retrovirus of the Friend retrovirus type,
b) a nucleotide sequence designated the *pol* sequence coding for polypeptides derived from a reverse transcriptase and an integrase (pol polypeptides) of a retrovirus of the Friend retrovirus type,
c) transcription regulatory signals controlling expression of the *gag* and *pol* sequences, and
d) a nucleotide sequence designated the *env* sequence, coding for polypeptides derived from the envelope (env polypeptides) of a retrovirus of the spumavirus family, the env polypeptides enabling encapsidation of retroviral RNAs,
e) transcription regulatory signals controlling expression of the *env* sequence.

6. Transcomplementing recombinant cell as claimed in claim 5, **characterised in that** it is the DOGOM1 cell registered with the CNCM *[Collection Nationale* de *Cultures* de *Micro-organismes -* National Collection of Microorganism Cultures] under the number 1-1496, recombined on the one hand with the gag and *pol* nucleotide sequences of the FB29 strain of the Friend retrovirus, under the control of the transcription signals contained in the LTR sequence of the FB29 strain of the Friend retrovirus, and recombined on the other hand with the *env* nucleotide sequence of a retrovirus of the spumavirus type, under the control of an inducible promoter, for example the RAR-β promoter.

7. Transcomplementing recombinant cell as claimed in claim 6, **characterised in that** it is the DOGOM1 cell registered with the CNCM under the number I-1496, recombined on the one hand with the *gag* and *pol* nucleotide sequences of the FB29 strain of the Friend retrovirus, under the control of a non-viral promoter, for example an inducible promoter such as the RAR-β promoter, or a conditional promoter.

8. Transcomplementing recombinant cell **characterised in that** it is a cell as claimed in any of claims 1 to 3, and **in that** it comprises:
a) a nucleotide sequence designated the gag sequence coding for polypeptides derived from a nucleoprotein (gag polypeptides) of a retrovirus of the Friend retrovirus type,
b) a nucleotide sequence designated the *pol* sequence coding for polypeptides derived from a reverse transcriptase and an integrase (pol polypeptides) of a retrovirus of the Friend retrovirus type,
c) transcription regulatory signals controlling expression of the *gag* and *pol* sequences, for example non-viral signals, and
d) a nucleotide sequence designated the *env* sequence, coding for polypeptides derived from an amphotrophic envelope, for example the 4070A envelope of the Moloney leukaemia virus (Mo-MuLV),
e) non-viral transcription regulatory signals, controlling expression of the *env* sequence, containing for example an inducible or conditional promoter, for example the RAR-β.

9. Transcomplementing recombinant cell as claimed in any of claims 4 to 8, **characterised in that** the nucleotide sequence of the expression vector for transcomplementation, coding for the envelope polypeptides is modified, for example by replacement of nucleotides by a sequence of nucleotides coding for a sequence of a polypeptide or a glycoprotein recognized specifically by a defined cell type, or by addition of such a sequence.

10. Transcomplementing recombinant cell as claimed in any of claims 4 to 9, **characterised in that** it is in addition transfected by a retroviral vector for the expression and/or the transfer and/or the integration within a target cell genome of a chosen nucleotide sequence ("transgenic sequence"), said retroviral vector being the pFOCH29 vector shown in figure 1 and registered with the CNCM under the number I-1326, in which the aforementioned transgenic sequence is inserted in a site not essential for its replication.

11. Transcomplementing recombinant cell as claimed in any of claims 5 to 9, **characterised in that** the retroviral vector is derived from the pFOCH29 vector registered with the CNCM under the number I-1326, in particular by deletion in the LTR sequence (pFOCH29PL vector shown in figure 2).

12. Transcomplementing recombinant cell as claimed in any of claims 4 to 11, **characterised in that** the transgenic sequence codes for a gene with therapeutic purposes, or for an antisense nucleotide sequence, or for a dominant negative mutant of a given gene or a sequence coding for a functional inhibitor of a given gene for a marker gene or for a sequence for regulation of a given gene or for a gene capable of adding a new function to the target cell.

## Patentansprüche

1. Eucaryont zur Enkapsidierung durch Transkomplementation rekombinanter retroviraler RNS zum Transfer und/oder zur Integration einer ausgewählten Nukleotidsequenz (transgene Sequenz) in ein Genom einer Zielzelle, wobei die besagte Zelle aus fötalen oder embryonalen Hundezellen ausgewählt wurde, so wie durch Selektion gemäß der folgenden Methode gewonnen:
a) Eucaryontenkultur, ausgewählt auf starkem ISCOVE-Medium (GIBCO), das unter anderem 10 % fötales Kälberserum und 10 % Pferdeserum enthält,
b) Übertragung der kultivierten Zellen in DMEM-Medium, das 20 % fötales Kälberserum enthält und Rückgewinnung ausgewählter Zellen und Zellkultur für einen Monat in einem Medium ohne CO₂,
c) Auswahl der Zellen mit einer hohen Teilungsrate (beschleunigte Kinetik),
d) Übertragung der ausgewählten Zellen in DMEM-Medium, das 10 % fötales Kälberserum enthält,
e) Übertragung der in Schritt d) zurückgewonnenen Zellen in DMEM-Medium, das 10 % Serum von neugeborenen Kälbern enthält (HyClone),
f) Rückgewinnung der nach Schritt e) ausgewählten Zellen und
die besagte Zelle ist **dadurch gekennzeichnet, dass** sie die folgenden Eiqenschaften bestätigt:
- sie hat eine homogene und zeitlich stabile Morphologie,
- ihr Ursprung ist kein Tumor,
- sie hat die Fähigkeit, in einem minimalen Kulturmedium ohne CO₂ ohne vorherige Transformation ausgewählt zu werden,
- sie hat eine hohe Zellteilungsrate.

2. Zelle nach Anspruch 1, **dadurch gekennzeichnet, dass** es sich um eine fötale Hundezelle DOGOM1 handelt, die am 30. November 1994 unter der Nummer I-1496 bei der CNCM hinterlegt wurde.

3. Rekombinante Zelle zur Enkapsidierung rekombinanter retroviraler RNA durch Transkomplementation (transkomplementante rekombinante Zelle), **dadurch gekennzeichnet, dass** es sich um eine Zelle nach Anspruch 1 oder 2 handelt, die zum Beispiel durch Transfektion oder Infektion mit den für die Transkomplementations-Polypeptide oder -Glykoproteine kodierenden Nukleotidsequenzen modifiziert wurde, wobei die besagten Sequenzen mit Hilfe von mindestens zwei Vektoren eingebracht wurden.

4. Rekombinante Zelle zur Enkapsidierung rekombinanter retroviraler RNA durch Transkomplementation (transkomplementante rekombinante Zelle), **dadurch gekennzeichnet, dass** es sich um eine Zelle nach einem der Ansprüche 1 oder 2 handelt, die genetisch modifiziert wurde zum Beispiel durch Transfektion oder Infektion mit:
1) einem ersten Expressionsvektor für die Komplementation in der Hülle eines retroviralen Vektors, der den Transfer und/oder die Integration einer ausgewählten Nukleotidsequenz (so genannte "transgene Sequenz") in das Genom einer Zielzelle ermöglicht, wobei der Vektor für die Komplementation eine für die Polypeptide der Hülle kodierende Nukleotidsequenz und Elemente zur Regulierung der Expression dieser Sequenz aufweist,
2) einem zweiten Expressionsvektor für die Transkomplementation eines retroviralen Vektors, der den Transfer und/oder die Integration einer ausgewählten Nukleotidsequenz (so genannte "transgene Sequenz") in das Genom einer Zielzelle ermöglicht, aufweisend:
a) eine Nukleotidsequenz, gag Sequenz genannt, die für die von einem Nukleoprotein (gag Polypeptide) eines Retrovirus vom Typ des Friend' schen Retrovirus abgeleiteten Polypeptide kodiert,
b) eine Nukleotidsequenz, *pol* Sequenz genannt, die für die von einem Retrovirus vom Typ des Friend'schen Retrovirus abgeleiteten Polypeptide, darunter ein inverses Transkriptase-Protein und eine Integrase (pol Polypeptide), kodiert,
c) regulierende Transkriptionssignale, die die Expression der Sequenzen *gag* und *pol* kontrollieren,
wobei der erste und der zweite Expressionsvektor keine Enkapsidierungssignale haben.

5. Transkomplementante rekombinante Zelle, **dadurch gekennzeichnet, dass** es sich um eine Zelle nach einem der Ansprüche 1 bis 3 handelt, und **dadurch**, dass sie aufweist:
a) eine Nukleotidsequenz, *gag* Sequenz genannt, die für die von einem Nukleoprotein (gag Polypeptide) eines Retrovirus vom Typ des Eriend' schen Retrovirus abgeleiteten Polypeptide kodiert,
b) eine Nukleotidsequenz, *pol* Sequenz genannt, die für die von einer inversen Transkriptase und einer Integrase (pol Polypeptide) eines Retrovirus vom Typ des Friend'schen Retrovirus abgeleiteten Polypeptide kodiert,
c) regulierende Transkriptionssignale, die die Expression der Sequenzen *gag* und *pol* kontrollieren, und
d) eine Nukleotidsequenz, *env* Sequenz genannt, die für die von der Hülle (*env* Polypeptide) eines Retrovirus aus der Familie der Spumaviren abgeleiteten Polypeptide kodieren, wobei die *env* Polypeptide die Enkapsidierung retroviraler RNA ermöglichen,
e) regulierende Transkriptionssignale, die die Expression der Sequenz *env* kontrollieren.

6. Transkomplementante rekombinante Zelle nach Anspruch 5, **dadurch gekennzeichnet, dass** es sich um die Zelle DOGOM1 handelt, die bei der CNCM unter der Nummer I-1496 hinterlegt wurde, die einerseits mit den Nuklcotidsequenzen gag und *pol* des Stamms FB29 des Friend'schen Retrovirus unter Kontrolle der in der Sequenz LTR des Stamms FB29 des Friend' schen Retrovirus enthaltenen Transkriptionssignale rekombiniert wurde, und andererseits mit der Nukleotidsequenz *env* eines Retrovirus von Typ eines Spumavirus unter der Kontrolle eines induzierbaren Promoters, zum Beispiel des Promoters RAR-β, rekombiniert wurde.

7. Transkomplementante rekombinante Zelle nach Anspruch 6, **dadurch gekennzeichnet, dass** es sich um die Zelle DOGOM1 handelt, die bei der CNCM unter der Nummer I-1496 hinterlegt wurde, die einerseits mit den Nukleotidsequenzen gag und *pol* des Stamms FB29 des Friend'schen Retrovirus unter Kontrolle eines nicht viralen Promoters, zum Beispiel eines induzierbaren Promoters, wie dem Promoter RAR-β, rekombiniert wurde, oder eines konditionellen Promoters.

8. Transkomplementante rekombinante Zelle, **dadurch gekennzeichnet, dass** es sich um eine Zelle nach einem der Ansprüche 1 bis 3 handelt, und **dadurch**, dass sie aufweist:
a) eine Nukleotidsequenz, gag Sequenz genannt, die für die von einem Nukleoprotein (gag Polypeptide) eines Retrovirus vom Typ des Friend'schen Retrovirus abgeleiteten Polypeptide kodiert,
b) eine Nukleotidsequenz, *pol* Sequenz genannt, die für die von inversen Transkriptase und einer Integrase (*pol* Polypeptide) eines Retrovirus vom Typ des Friend' schen Retrovirus abgeleiteten Polypeptide kodiert,
c) regulierende Transkriptionssignale, die die Expression der Sequenzen *gag* und *pol* kontrollieren, zum Beispiel nicht virale Signale, und
d) eine Nukleotidsequenz, *env* Sequenz genannt, die für die von einer amphotropen Hülle, zum Beispiel der Hülle 4070A des Moloney-Leukämievirus (Mo-MuLV), abgeleiteten Polypeptide kodieren,
e) nicht virale regulierende Transkriptionssignale, die die Expression der Sequenz *env* kontrollieren, die zum Beispiel einen induzierbaren oder konditionellen Promoter, zum Beispiel den Promoter RAR-β, enthalten.

9. Transkomplementante rekombinante Zelle nach einem der Ansprüche 4 bis 8, **dadurch gekennzeichnet, dass** die Nukleotidsequenz des Expressionsvektors für die Transkomplementation, die für die Polypeptide der Hülle kodiert, modifiziert wird, zum Beispiel durch Ersetzen von Nukleotiden durch eine Kette von Nukleotiden, die für eine Sequenz eines Polypeptids oder eines Glykoproteins, das speziell von einen bestimmten Zelltyp erkannt wird, kodiert, oder durch Hinzufügen einer derartigen Kette.

10. Transkomplementante rekombinante Zelle nach einem der Ansprüche 4 bis 9, **dadurch gekennzeichnet, dass** sie unter anderem von einem retroviralen Vektor zur Expression und/oder dem Transfer und/oder die Integration einer ausgewählten Nukleotidsequenz ("transqene Sequenz") in ein Genom einer Zielzelle transfiziert wird, wobei der besagte retrovirale Vektor der Vektor pFOCH29 ist, der in Fig. 1 dargestellt wird und bei der CNCM unter der Nr. 1-1326 hinterlegt wurde, in den an einem für seine Replikation nicht wesentlichen Ort die oben genannte transgene Sequenz eingeführt wurde.

11. Transkomplementante rekombiante Zelle nach einem der Ansprüche 5 bis 9, **dadurch gekennzeichnet, dass** der retrovirale Vektor vom bei der CNCM unter der Nr. 1-1326 hinterlegten Vektor pFOCH29 abgeleitet wurde, vor allem durch Deletion auf Ebene der Sequenz LTR (Vektor pFOCH29PL, auf Fig. 2 dargestellt).

12. Transkomplementante rekombinante Zelle nach einem der Ansprüche 4 bis 11, **dadurch gekennzeichnet, dass** die transgene Sequenz für ein Gen mit therapeutischem Ziel kodiert, oder für eine Antisens-Nukleotidsequenz, oder für einen dominanten negativen Mutanten eines bestimmten Gens oder eine Sequenz, die für einen funktionellen Inhibitor eines bestimmten Gens für ein Markergen kodiert, oder für eine Regulationssequenz eines bestimmten Gens oder für ein Gen, das der Zielzelle eine neue Funktion hinzufügen könnte.
